# EUROPEAN PATENT APPLICATION

(11) **EP 3 834 738 A2**
(43) Date of publication of application: **16.06.2021**
(21) Application number: 20193071.6
(22) Date of filing: 27.08.2020
(51) Int. Cl.: A61B 17/00

(54) **EROSION MITIGATING OCCLUDER**

(30) Priority: 27.08.2019 US 201962892140 P; 06.08.2020 US 202063062083 P
(71) Applicant: St. Jude Medical, Cardiology Division, Inc., St. Paul MN 55117-9913 (US)
(72) Inventor: MEYER, Michael P., Minnestrina, MN Minnesota 55359 (US); GUTFINGER, Dan E., Agoura Hills, CA California 93201 (US); GUPTA, Pankaj, Maple Grove, MN Minnesota 55311 (US); PERSZYK, Brian, Shoreview, MN Minnesota 55126 (US); BLOOMQUIST, Alex, Mound, MN Minnesota 55364 (US); WILLIAMS, Hanna, St. Louis Park, MN Minnesota 55426 (US); BEEK, Erika, Bloomington, MN Minnesota 55437 (US)
(74) Representative: Savoca, Agatino

(57) **Abstract**

The present disclosure is directed to embodiments and methods of reducing or eliminating erosion resulting from the use of an occluder. In particular, the present disclosure is directed to reducing or eliminating erosion resulting from the use of an occluder while maintaining the fundamental function and effectiveness of the occluder with an improved occluder braid pattern. The embodiments and methods disclosed herein reduce or eliminate erosion, for example, by reducing the friction and force of an occluder on cardiac tissue and/or by increasing occluder disc compliance to cardiac structures and movement.

## Description

### CROSS-REFERENCE TO RELATED APPLICATIONS

The present application claims the benefit of priority to U.S. Provisional Patent Application No. 62/892,140, filed August 27, 2019, and to U.S. Provisional Patent Application No. 63/062,083, filed August 6, 2020, the entire contents and disclosure of which are hereby incorporated by reference herein.

### BACKGROUND OF THE DISCLOSURE

### A. Field of the Disclosure

The present disclosure relates generally to medical devices that are used in the human body. In particular, the present disclosure is directed to embodiments and methods of reducing or eliminating erosion resulting from the use of an occluder. More specifically, the present disclosure is directed to reducing or eliminating erosion while maintaining the fundamental function and effectiveness of an occluder with an improved occluder braid pattern. The embodiments and methods disclosed herein reduce or eliminate erosion, for example, by reducing the friction or force of an occluder on cardiac tissue and/or by increasing occluder disc compliance to cardiac structures and movements.

### B. Background

An occluder is a device used in trans-catheter secundum atrial septal defect closures. Secundum atrial septal defects are common congenital heart defects that allow blood to flow between the left and right atria of the heart, thus decreasing cardiac output and increasing the workload on the heart. Occluders are generally delivered through a sheath in the femoral vein and deployed in the defect to occlude blood flow.

A rare, but adverse event that has been reported to occur in some occluder implantations is erosion of the atrial wall tissue. Erosion is a wearing away of the tissue due to the friction between the occluder and the tissue. The result of this tissue erosion can be removing the device, fixing eroded holes and/or surgically closing defects.

The friction force can be lowered by making the occluder softer (e.g., more compliant), however, a softer device is more prone to bulging into the atrium(s) as forces are applied to the waist and discs of the occluder. A common problem with softer devices (e.g., devices having a softer braided layer formed by using a smaller wire diameter, differing number of wires, and/or alternate braid pattern as compared to stiffer devices) is a lack of radial strength in the waist of the device. When deployed in thick and or asymmetric septa, the softness and conformability of these softer devices cause the braid to elongate, which causes bulging of the discs and shrinkage in the waist. Waist shrinkage results in inadequate filling of the defect by the waist of the device and additionally creates pathways for leak across the device.

Accordingly, it would be desirable to reduce or eliminate erosion of cardiac tissue while maintaining the fundamental function and effectiveness of an occluder. It would be further desirable to increase the radial strength at the waist of the device without stiffening or altering the device as a whole, to allow the discs and disc edges to retain enough softness/compliance to be conformable while still filling and sealing the defect.

### BRIEF SUMMARY OF THE DISCLOSURE

The present disclosure generally relates to reducing and/or eliminating erosion of cardiac tissue while maintaining the fundamental function and effectiveness of an occluder. The present disclosure discloses devices and methods to accomplish this objective, through, for example, reducing friction and force on cardiac tissue and/or by increasing the occluder device compliance to cardiac structures and movements.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site. The medical device also includes a fabric coating covering at least one of the proximal disc portion and the distal disc portion.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site. The medical device also includes a fabric coating covering at least one of the proximal disc portion and the distal disc portion. The method also includes constraining the medical device from a preset expanded configuration to a reduced configuration; delivering the medical device; deploying the medical device such that the tubular member returns to the preset expanded configuration; and, eliminating or reducing friction of the medical device on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer is encapsulated with a polymer coating.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer is encapsulated with a polymer coating; constraining the medical device from a preset expanded configuration to a reduced configuration; delivering the medical device; deploying the medical device such that the tubular member returns to the preset expanded configuration; and eliminating or reducing friction of the medical device on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, and wherein a parylene coating covers at least a portion of the at least one braided layer.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, and wherein a parylene coating covers at least a portion of the at least one braided layer; constraining the medical device from a preset expanded configuration to a reduced configuration; delivering the medical device; deploying the medical device such that the tubular member returns to the preset expanded configuration; and, eliminating or reducing friction of the medical device on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer comprises a polymeric fabric coating located on an outside surface of the braided layer, wherein the polymeric fabric coating is deposited on the outside surface of the braided layer through an electrospinning process.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer comprises a polymeric fabric coating located on an outside surface of the braided layer, wherein the polymeric fabric coating is deposited on the outside surface of the braided layer through an electrospinning process; constraining the medical device from a preset expanded configuration to a reduced configuration; delivering the medical device; deploying the medical device such that the tubular member returns to the preset expanded configuration; and, eliminating or reducing friction of the medical device on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, wherein the at least one braided layer comprises a ceramic coating on an outside surface of the braided layer.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, wherein the at least one braided layer comprises a ceramic coating on an outside surface of the braided layer; constraining the medical device from a preset expanded configuration to a reduced configuration; delivering the medical device; deploying the medical device such that the tubular member returns to the preset expanded configuration; and, eliminating or reducing friction of the medical device on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer comprises a wire braid design between a 72 wire braid design and a 288 wire braid design, including all wire braid designs therebetween.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer comprises a wire braid design between a 72 wire braid design and a 288 wire braid design, including all wire braid designs therebetween; constraining the medical device from a preset expanded configuration to a reduced configuration; delivering the medical device; deploying the medical device such that the tubular member returns to the preset expanded configuration; and, increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises multiple braided layers, wherein each braided layer comprises a unique layer geometry relative to the other braided layers of the multiple braided layers.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises multiple braided layers, wherein each braided layer comprises a unique layer geometry relative to the other braided layers of the multiple braided layers; constraining the medical device from a preset expanded configuration to a reduced configuration; delivering the medical device; deploying the medical device such that the tubular member returns to the preset expanded configuration; and, increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer with material removed from a portion thereof, wherein the portion of the braided layer with material removed comprises a smaller braid wire diameter at the proximal disc portion and the distal disc portion than at the waist member.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer with material removed from a portion thereof, wherein the portion of the braided layer with material removed comprises a smaller braid wire diameter at the proximal disc portion and the distal disc portion than at the waist member; constraining the medical device from a preset expanded configuration to a reduced configuration; delivering the medical device; deploying the medical device such that the tubular member returns to the preset expanded configuration; and, increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, wherein the tubular member further comprises a proximal transition segment and a distal transition segment, wherein the proximal transition segment connects the proximal disc portion to the waist member and the distal transition segment connects the distal disc portion to the waist member, and further wherein each of the proximal transition segment and the distal transition segment has a smaller diameter than the waist member.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, wherein the tubular member further comprises a proximal transition segment and a distal transition segment, wherein the proximal transition segment connects the proximal disc portion to the waist member and the distal transition segment connects the distal disc portion to the waist member, and further wherein each of the proximal transition segment and the distal transition segment has a smaller diameter than the waist member; constraining the medical device from a preset expanded configuration to a reduced configuration; delivering the medical device; deploying the medical device such that the tubular member returns to the preset expanded configuration; and, increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the proximal disc portion and the distal disc portion comprise an edge geometry selected from the group consisting of a tapered shape, a cup shape, and a round shape, and further wherein the tubular member comprises at least one braided layer and has an expanded configuration when deployed at the target site and a reduced configuration for delivery to the target site.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the proximal disc portion and the distal disc portion comprise an edge geometry selected from the group consisting of a tapered shape, a cup shape, and a round shape, and further wherein the tubular member comprises at least one braided layer and has an expanded configuration when deployed at the target site and a reduced configuration for delivery to the target site; constraining the medical device from a preset expanded configuration to a reduced configuration; delivering the medical device; deploying the medical device such that the tubular member returns to the preset expanded configuration; and, increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member comprises at least one braided layer comprises a non-circular braid design, and wherein the tubular member has an expanded configuration when deployed at the target site and a reduced configuration for delivery to the target site.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member comprises at least one braided layer comprises a non-circular braid design, and wherein the tubular member has an expanded configuration when deployed at the target site and a reduced configuration for delivery to the target site; constraining the medical device from a preset expanded configuration to a reduced configuration; delivering the medical device; deploying the medical device such that the tubular member returns to the preset expanded configuration; and, increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the braided layer comprises multiple wire sizes.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the braided layer comprises multiple wire sizes; constraining the medical device from a preset expanded configuration to a reduced configuration; delivering the medical device; deploying the medical device such that the tubular member returns to the preset expanded configuration; and, increasing the medical device compliance on cardiac tissue.

In yet another embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, and wherein the waist member comprises a skirt coupled thereto.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, and wherein the waist member comprises a skirt coupled thereto; constraining the medical device from a preset expanded configuration to a reduced configuration; delivering the medical device; deploying the medical device such that the tubular member returns to the preset expanded configuration; and, increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein the at least one braided layer has a braid diameter greater than a diameter of a largest portion of the medical device.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein the at least one braided layer has a braid diameter greater than a diameter of a largest portion of the medical device. The method further comprises constraining the medical device in a reduced configuration, delivering the medical device, deploying the medical device such that the tubular member transitions from the reduced configuration to an expanded configuration, and increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein a first portion of the at least one braided layer covers the waist member and has a different braid pattern than a second portion of the at least one braided layer covering at least one of the proximal disc portion and the distal disc portion.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein a first portion of the at least one braided layer covers the waist member and has a different braid pattern than a second portion of the at least one braided layer covering at least one of the proximal disc portion and the distal disc portion. The method further comprises constraining the medical device in a reduced configuration, delivering the medical device, deploying the medical device such that the tubular member transitions from the reduced configuration to an expanded configuration, and increasing the medical device compliance on cardiac tissue.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein a pics per inch of the at least one braided layer changes along a length of the medical device.

In another embodiment, the present disclosure is directed to a method of eliminating or reducing erosion of cardiac tissue. The method comprises providing a medical device comprising a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein a pics per inch of the at least one braided layer changes along a length of the medical device. The method further comprises constraining the medical device in a reduced configuration, delivering the medical device, deploying the medical device such that the tubular member transitions from the reduced configuration to an expanded configuration, and increasing the medical device compliance on cardiac tissue.

The foregoing and other aspects, features, details, utilities and advantages of the present disclosure will be apparent from reading the following description and claims, and from reviewing the accompanying drawings.

### BRIEF DESCRIPTION OF THE DRAWINGS

FIG. 1 is an exemplary embodiment of a medical device in accordance with the present disclosure.
FIG. 2A is an exemplary embodiment of an occluder with a fabric coating on the disc edges in accordance with the present disclosure. FIG. 2B is an exemplary embodiment of an occluder with a fabric coating completely covering the discs in accordance with the present disclosure. FIG. 2C is an exemplary embodiment of an occluder with a fabric coating covering both the discs and the waist in accordance with the present disclosure. FIG. 2D is an exemplary embodiment of an occluder with a fabric coating covering the entire occluder in accordance with the present disclosure.
FIGs. 3A and 3B depict exemplary embodiments of braid encapsulation coatings in accordance with the present disclosure.
FIG. 4A is an exemplary embodiment of a fully covered waist member using a single section of covering/coating in accordance with the present disclosure. FIG. 4B and FIG. 4C are exemplary embodiments of a fully covered waist member using multiple sections of covering/coating in accordance with the present disclosure. FIG. 4D and FIG. 4E are exemplary embodiments of a partially covered waist member using multiple sections of covering/coating in accordance with the present disclosure.
FIG. 5 illustrates an exemplary embodiment of an electrospinning process performed to coat a braid in accordance with the present disclosure.
FIG. 6A is an exemplary embodiment of an open-center braided occluder with a braid having a braid diameter equal to a diameter of a distal disc portion of the occluder. FIG. 6B is an enlarged view of the occluder shown in FIG. 6A. FIG. 6C is an exemplary embodiment of a closed-center braided occluder with a braid having a braid diameter equal to a diameter of a distal disc portion of the occluder. FIG. 6D is an enlarged view of the occluder shown in FIG. 6C.
FIG. 7A is an exemplary embodiment of another open-center braided occluder having a braid diameter greater than a distal disc portion of the occluder, in accordance with the present disclosure. FIG. 7B is an enlarged view of the occluder shown in FIG. 7A. FIG. 7C is an exemplary embodiment of another closed-center braided occluder with a braid having a braid diameter equal to a diameter of a distal disc portion of the occluder. FIG. 7D is an enlarged view of the occluder shown in FIG. 7C.
FIGs. 8A and 8B are side views of the braided occluders shown in FIGs. 6A and 7A, respectively, illustrating a bulging comparison between the two occluders.
FIG. 9A is an exemplary embodiment of a braided occluder having a standard waist braid pattern. FIG. 9B is an enlarged view of the waist braid pattern shown in FIG. 9A.
FIG. 10A is an exemplary embodiment of a tapered braided occluder with a relatively low pics per inch (PPI) waist braid pattern in accordance with the present disclosure. FIG. 10B is an enlarged view of the waist braid pattern shown in FIG. 10A.
FIG. 11 is a graph illustrating waist compression forces of different braided occluders in accordance with the present disclosure.
FIG. 12A illustrates an exemplary embodiment of a braid pattern on a mandrel where the braid pattern has a changing or variable pic rate across the length of the braid in accordance with the present disclosure. FIG. 12B illustrates an enlarged view of the braid pattern shown in FIG. 12A.

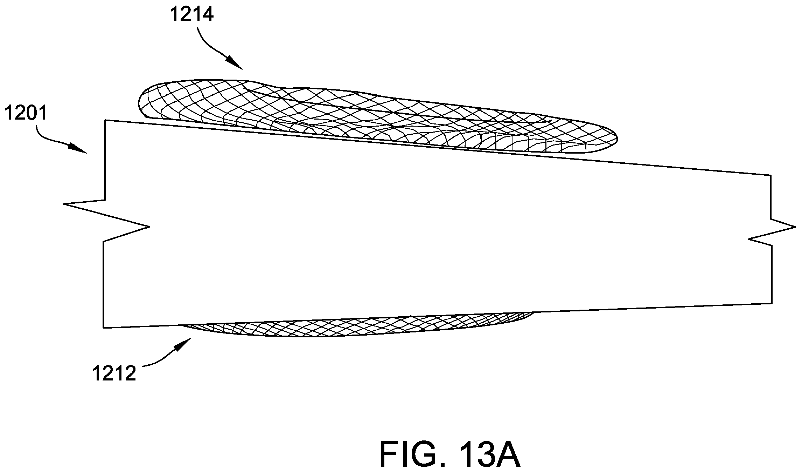

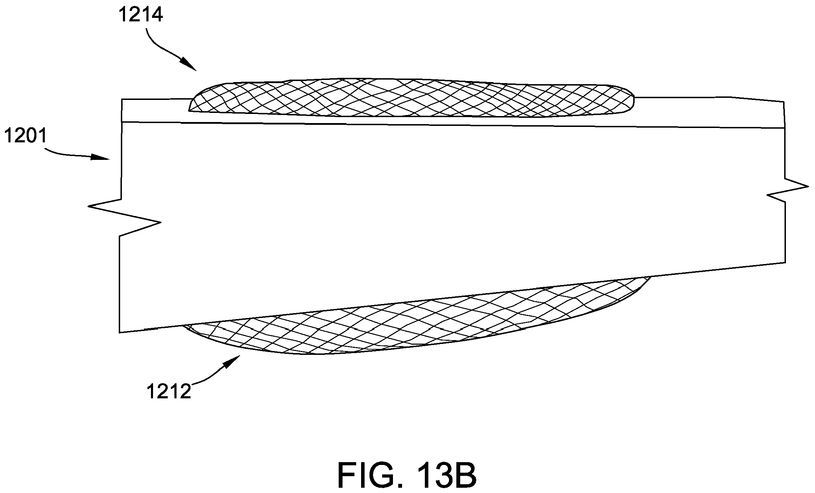

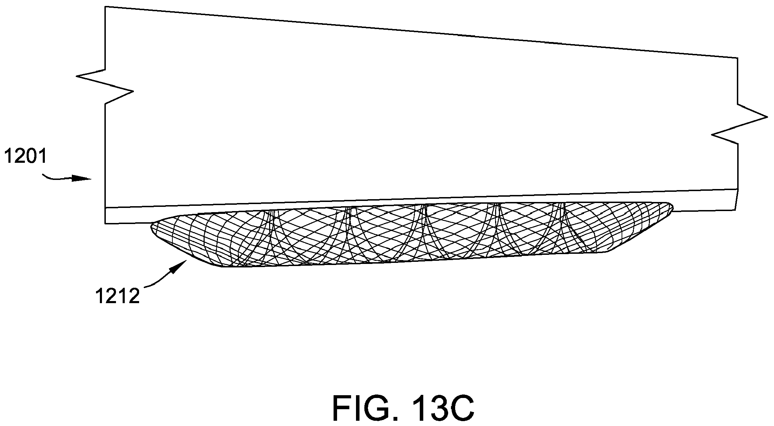

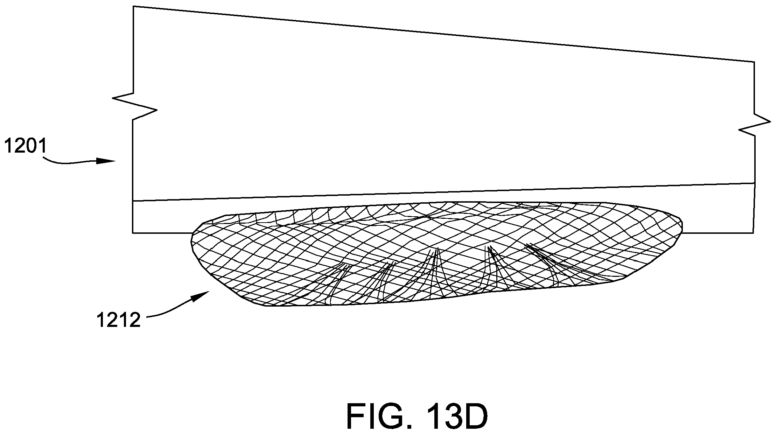

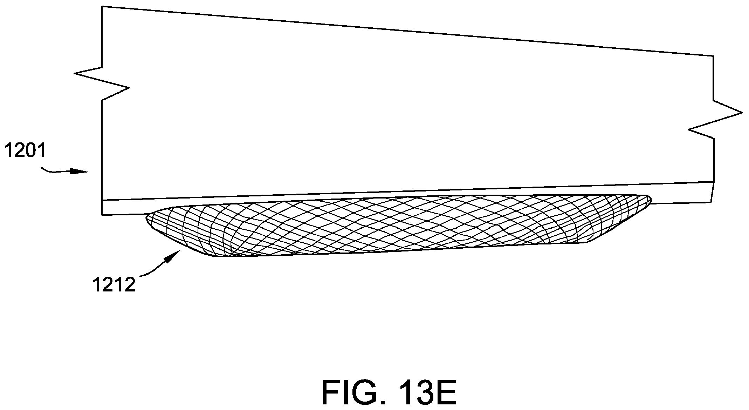

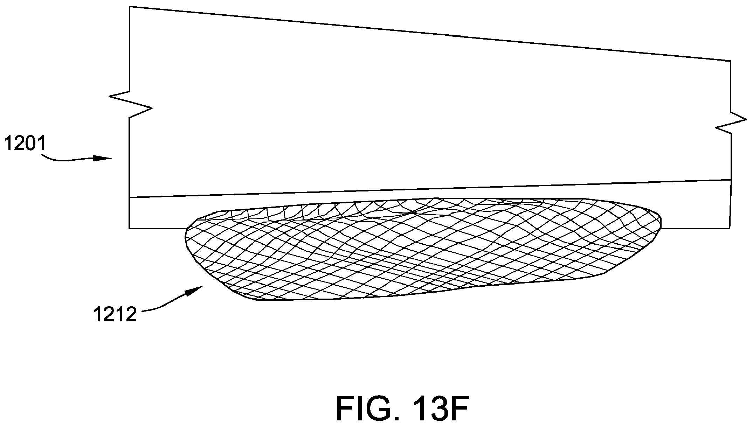

FIG. 13A illustrates an exemplary embodiment of a transitioned braided occluder having a braid pattern with a changing pic rate in a thick septum and with increased conformance (and less bulging) of the proximal disc (in comparison with the proximal disc of FIG. 13B) in accordance with the present disclosure. FIG. 13B illustrates an exemplary embodiment of a standard braided occluder having a braid pattern with a generally consistent pic rate in a thick septum and with increased bulging (and less conformance) of the proximal disc (in comparison with the proximal disc of FIG. 13A) in accordance with the present disclosure. FIG. 13C illustrates an exemplary embodiment of an open-center transitioned braided occluder having a braid pattern with a changing pic rate in a thick septum and with increased conformance (and less bulging) of the distal disc (in comparison with the distal disc of FIG. 13D) in accordance with the present disclosure. FIG. 13D illustrates an exemplary embodiment of an open-center standard braided occluder having a braid pattern with a generally consistent pic rate in a thick septum and with increased bulging (and less conformance) of the distal disc (in comparison with the distal disc of FIG. 13C) in accordance with the present disclosure. FIG. 13E illustrates an exemplary embodiment of a closed-center transitioned braided occluder having a braid pattern with a changing pic rate in a thick septum and with increased conformance (and less bulging) of the distal disc (in comparison with the distal disc of FIG. 13F) in accordance with the present disclosure. FIG. 13F illustrates an exemplary embodiment of a closed-center standard braided occluder having a braid pattern with a generally consistent pic rate in a thick septum and with increased bulging (and less conformance) of the distal disc (in comparison with the distal disc of FIG. 13E) in accordance with the present disclosure.
FIG. 14A illustrates an exemplary embodiment of a septum cutaway of a transitioned braided occluder with a changing pic rate in accordance with the present disclosure. FIG. 14B illustrates an exemplary embodiment of a septum cutaway of a standard braided occluder with a generally consistent pic rate in accordance with the present disclosure.

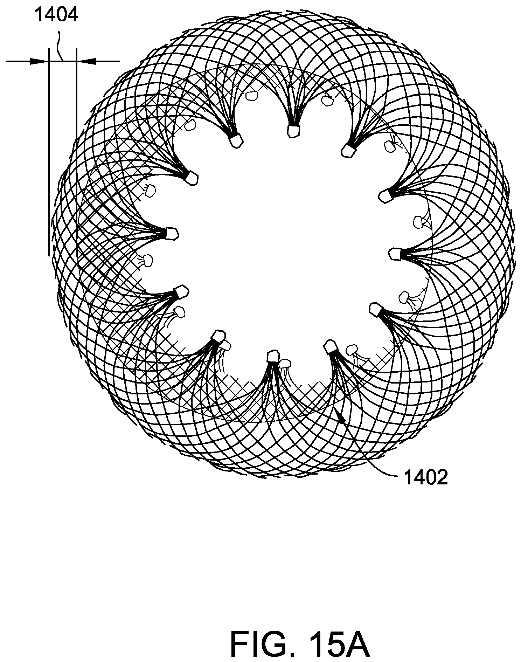

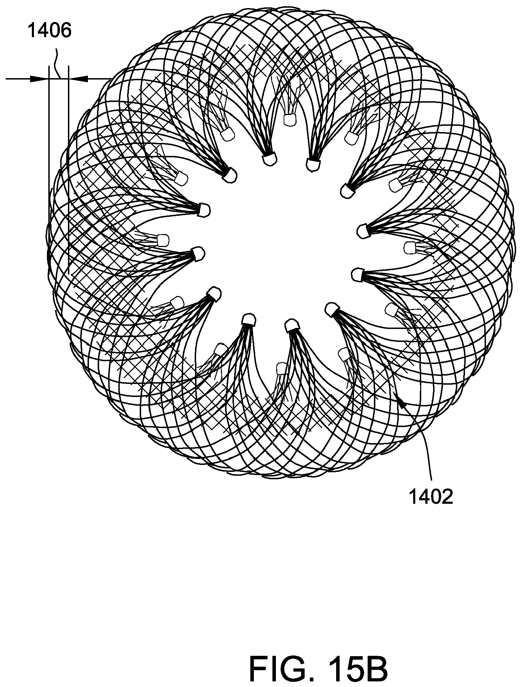

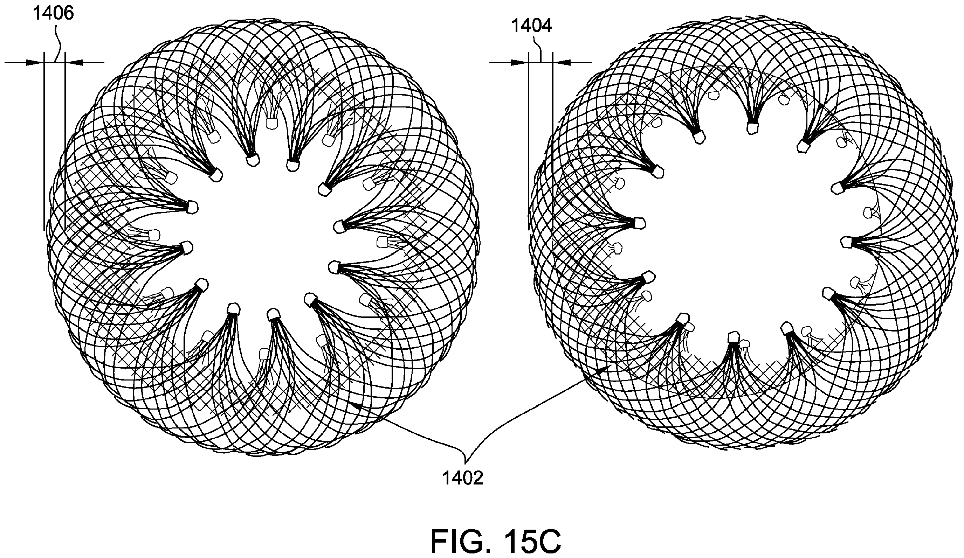

FIG. 15A illustrates an exemplary embodiment of a top view of a transitioned braided occluder with a changing pic rate, with view from the proximal end of the device showing proximal disc coverage of the defect in accordance with the present disclosure. FIG. 15B illustrates an exemplary embodiment of a top view of a standard braided occluder with a generally consistent pic rate, with view from the proximal end of the device showing proximal disc coverage of the defect in accordance with the present disclosure. FIG. 15C illustrates a side-by-side view of the braid patterns shown in FIG. 15A (left) and FIG. 15B (right).

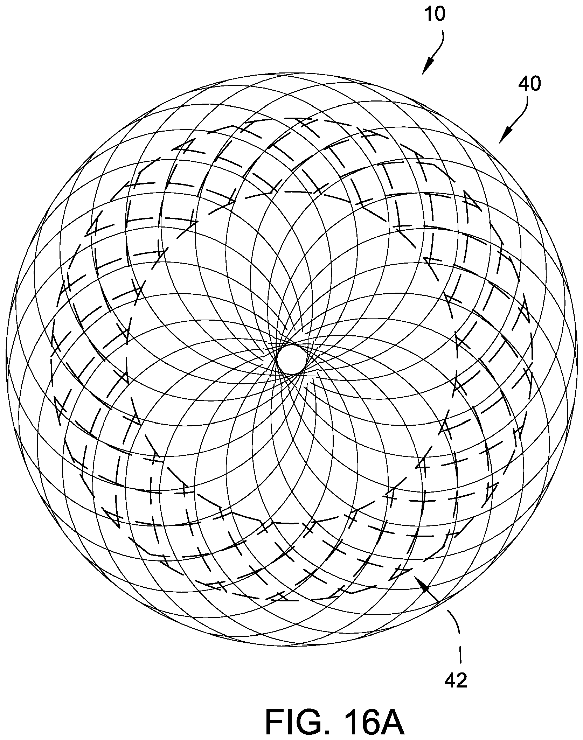

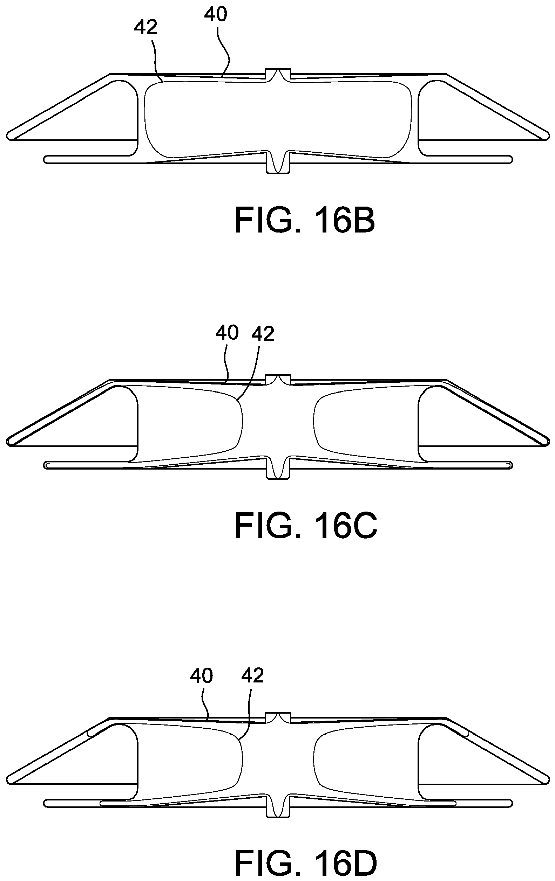

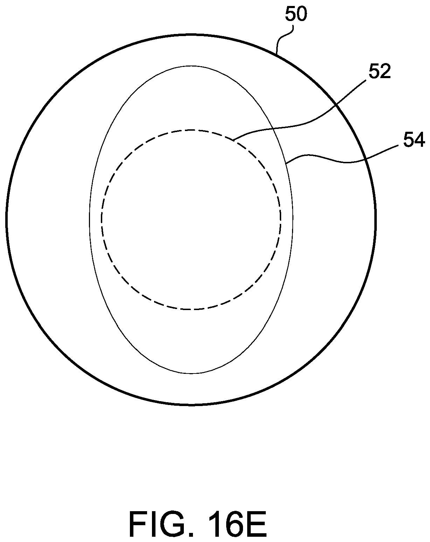

FIGs. 16A and 16B depict exemplary embodiments of an occluder configuration that has two layers with different diameter discs in accordance with the present disclosure. FIG. 16C is an exemplary embodiment of an occluder configuration with the inner layer braid matching the disc diameters of the outer layer but with a waist of the inner layer having a smaller diameter than the outer layer in accordance with the present disclosure. FIG. 16D is an exemplary embodiment of an occluder configuration with an inner layer having discs and a waist with smaller diameters than the outer layer in accordance with the present disclosure. FIG. 16E is an exemplary embodiment of an occluder configuration in accordance with the present disclosure.
FIG. 17 depicts an exemplary embodiment of an occluder configuration having an additional fold incorporated in the braided layer at the waist of the device in accordance with the present disclosure.
FIGs. 18A and 18B depict an exemplary embodiment of an occluder configuration having a varying braid wire thickness in accordance with the present disclosure.
FIGs. 19A and 19B depict exemplary embodiments of occluder configurations having thinner disc waists in accordance with the present disclosure.
FIGs. 20A, 20B and 20C depict exemplary embodiments of occluder configurations that are currently used.
FIGs. 21A and 21B depict an exemplary embodiment of an occluder configuration having rounded disc edges in accordance with the present disclosure. FIG. 21C is an exemplary embodiment of an occluder configuration having tapered-shaped disc edges in accordance with the present disclosure. FIG. 21D is an exemplary embodiment of an occluder configuration having hourglass-shaped disc edges in accordance with the present disclosure. FIG. 21E is an exemplary embodiment of an occluder configuration having cup-shaped disc edges in accordance with the present disclosure.

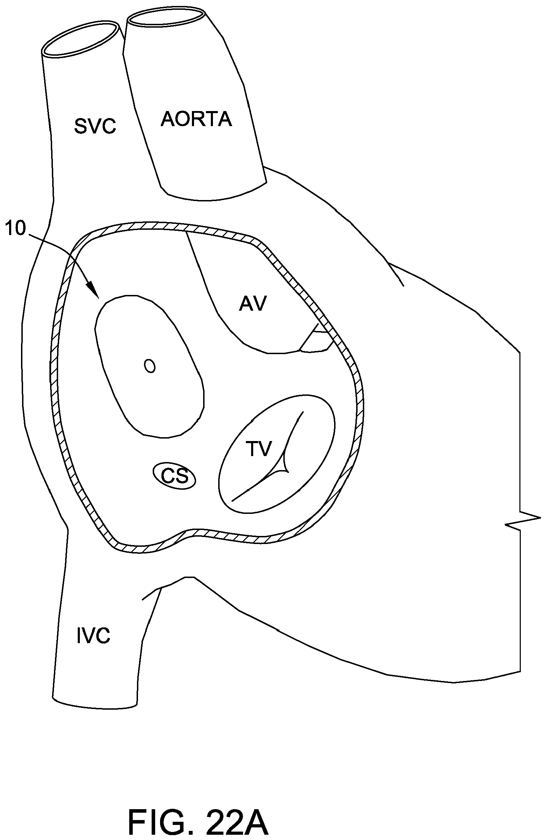

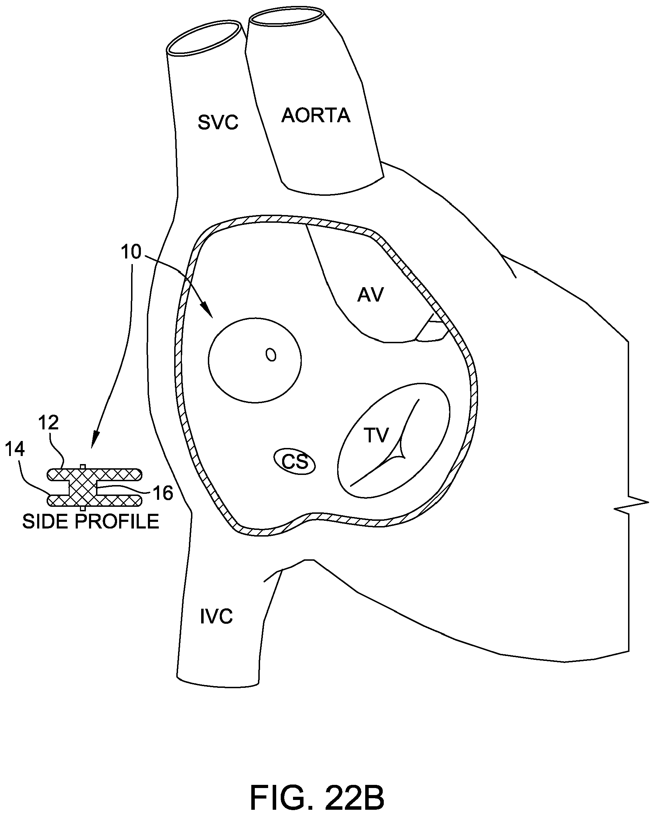

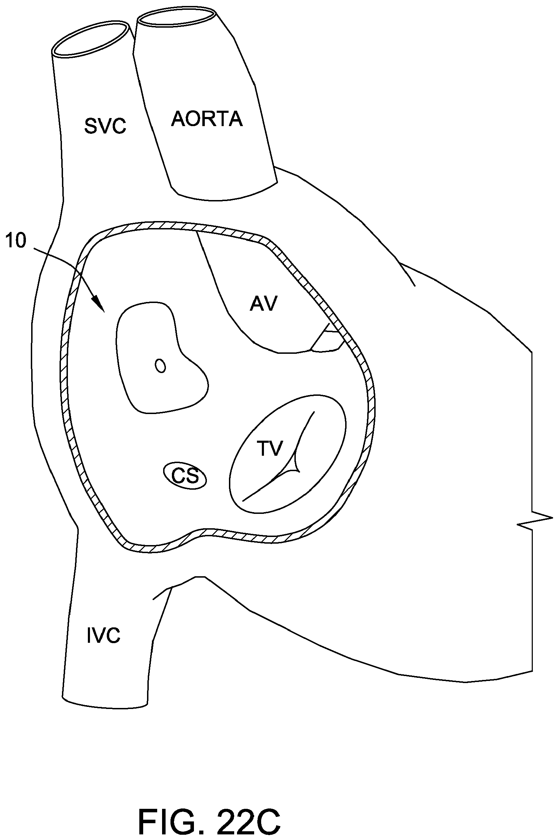

FIG. 22A is an exemplary embodiment of an occluder configuration having a non-circular braid design with an oval shape, shown in an operational state implanted in a patient's heart, in accordance with the present disclosure. FIG. 22B is an exemplary embodiment of an occluder configuration having a non-circular braid design with offset discs, shown in an operational state implanted in a patient's heart, in accordance with the present disclosure. FIG. 22C is an exemplary embodiment of an occluder configuration having a non-circular braid design where the braid enters on the side that is towards the aorta such that it can go around or saddle the aorta in accordance with the present disclosure.
FIGs. 23A and 23B are exemplary embodiments of disc profiles in accordance with the present disclosure.
FIG. 24 is an exemplary embodiment of a termination profile in accordance with the present disclosure.
FIG. 25A is an exemplary embodiment of a non-self-centering occluding device. FIG. 23B is an exemplary embodiment of a self-centering occluding device.
FIG. 26A is an exemplary embodiment of an occluder including a skirt in accordance with the present disclosure. FIG. 26B is a profile view of an exemplary embodiment of an occluder including a skirt in accordance with the present disclosure.
FIG. 27A is an exemplary embodiment of an occluder including a skirt covering the edge of a disc in accordance with the present disclosure. FIG. 27B is a profile view of FIG. 27A. FIG. 27C is an exemplary embodiment of an occluder including a skirt covering the edge of multiple discs in accordance with the present disclosure. FIG. 27D is a profile view of FIG. 27C.
FIG. 28 is an exemplary embodiment of an occluding device in accordance with the present disclosure.
FIG. 29 is an exemplary embodiment of an occluding device in accordance with the present disclosure.
FIG. 30 is an exemplary embodiment of an occluding device in accordance with the present disclosure.

Corresponding reference characters indicate corresponding parts throughout the several views of the drawings. It is understood that that Figures are not necessarily to scale.

### DETAILED DESCRIPTION OF THE DISCLOSURE

The present disclosure relates generally to medical devices that are used in the human body. In particular, the present disclosure generally relates to reducing and/or eliminating erosion of cardiac tissue while maintaining the fundamental function and effectiveness of an occluder. The present disclosure discloses devices and methods to accomplish this objective, through, for example, reducing friction and force on cardiac tissue and/or by increasing the occluder device compliance to cardiac structures and movements.

The disclosed embodiments may lead to more consistent and improved patient outcomes. It is contemplated, however, that the described features and methods of the present disclosure as described herein may be incorporated into any number of systems as would be appreciated by one of ordinary skill in the art based on the disclosure herein.

The present disclosure now will be described more fully hereinafter with reference to the accompanying drawings, in which some, but not all embodiments of the disclosure are shown. Indeed, this disclosure may be embodied in many different forms and should not be construed as limited to the embodiments set forth herein; rather, these embodiments are provided so that this disclosure will satisfy applicable legal requirements. Like numbers refer to like elements throughout.

Some embodiments of the present disclosure provide a medical device, such as an occlusion device (occluder), for use in occluding an abnormality in a patient's body, such as an Atrial Septal Defect (ASD), a Ventricular Septal Defect (VSD), a Patent Ductus Arteriosus (PDA), a Patent Foramen Ovale (PFO), conditions that result from previous medical procedures such as Para-Valvular Leaks (PVL) following surgical valve repair or replacement, and the like. The device may also be used as a flow restrictor or an aneurysm bridge or other type of occluder for placement in the vascular system. It is understood that the use of the term "abnormality" is not meant to be limiting, as the device may be configured to occlude any vessel, organ, opening, chamber, channel, hole, cavity, or the like, located anywhere in the body.

Some embodiments of the present disclosure provide an improved percutaneous catheter directed intravascular occlusion device for use in the vasculature in patients' bodies, such as blood vessels, channels, lumens, a hole through tissue, cavities, and the like, such as a PDA. Other physiologic conditions in the body occur where it is also desirous to occlude a vessel or other passageway to prevent blood flow into or therethrough. These device embodiments may be used anywhere in the vasculature where the anatomical conditions are appropriate for the design.

The medical device may include one or more layers of occlusive material, wherein each layer may be comprised of any material that is configured to substantially preclude or occlude the flow of blood so as to facilitate thrombosis. As used herein, "substantially preclude or occlude flow" shall mean, functionally, that blood flow may occur for a short time, but that the body's clotting mechanism or protein or other body deposits on the occlusive material results in occlusion or flow stoppage after this initial time period. For instance, occlusion may be clinically represented by injecting a contrast media into the upstream lumen of the device and if little or no contrast media flows through the device wall after a predetermined period of time, then the position and occlusion of the device is adequate as would be recognized by one of ordinary skill in the art. More specifically, the time for occlusion could begin after deployment of the medical device, such as after the device has expanded and engaged the lumen and the delivery device has been disconnected, until no contrast media (as observed with fluoroscopy) flows through the device. For instance, if the medical device is implanted within a lumen and contrast media is injected on one side of the device (e.g., a high pressure side) but no contrast media is observed on the opposite side of the device (e.g. a low pressure side), then the device has substantially precluded or occluded blood flow through the device. Thus, if the medical device is implanted within a PDA and contrast media is injected into the aorta and does not flow through the device to the pulmonary artery or remains stagnant within the device, then flow through the PDA is substantially precluded or occluded. According to one embodiment of the present disclosure, the device is configured to occlude at least a portion of a vessel, a channel, a lumen, an opening, or a cavity in less than about 10 minutes and even less than about 5 minutes with observed occlusions in testing as low as within about 1 minute. Thus, in one embodiment, there is not "immediate occlusion," as the device does not immediately obstruct all blood flow but, rather, the device slows the flow of blood in order for occlusion to occur as described above. Such immediate occlusion may result in problems in fixation or positioning of the device in the lumen or may result in suction or the complete stoppage of flow which may be undesirable in some circumstances.

### Reduction and/or Elimination of Friction and Force on Cardiac Tissue

In some embodiments of the present disclosure, the medical devices disclosed herein reduce and/or eliminate erosion of cardiac tissue while maintaining the fundamental function and effectiveness of an occluder (such as, for example, an Amplatzer™ Septal Occluder (ASO)). The medical devices achieve this objective by reducing friction and/or force of the device on cardiac tissue.

By decreasing the device friction and force on cardiac tissue, the device is less likely to produce significant wear, which results in erosion of the tissue. Through, for example, reduction of the coefficient of friction of the embodiments disclosed herein, the frictional interaction between the medical device and tissue will decrease and thus the likelihood of erosion is also reduced and/or eliminated.

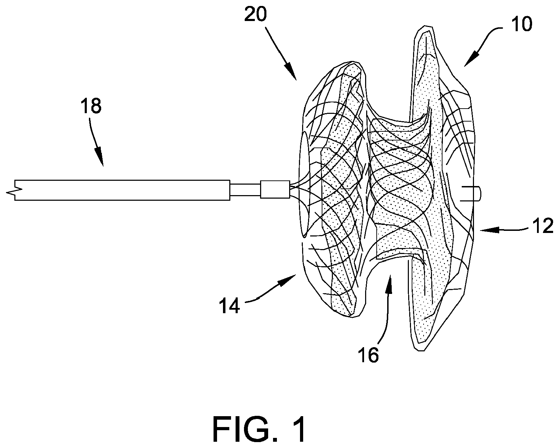

FIG. 1, by way of example, is an exemplary embodiment of a medical device 10 in accordance with the present disclosure. The device 10 (here, shown as an occluder) includes a left atrial disc 12 and a right atrial disc 14 connected by a waist member 16. A delivery cable 18 is connected to the device 10. The device 10 includes a braided layer 20.

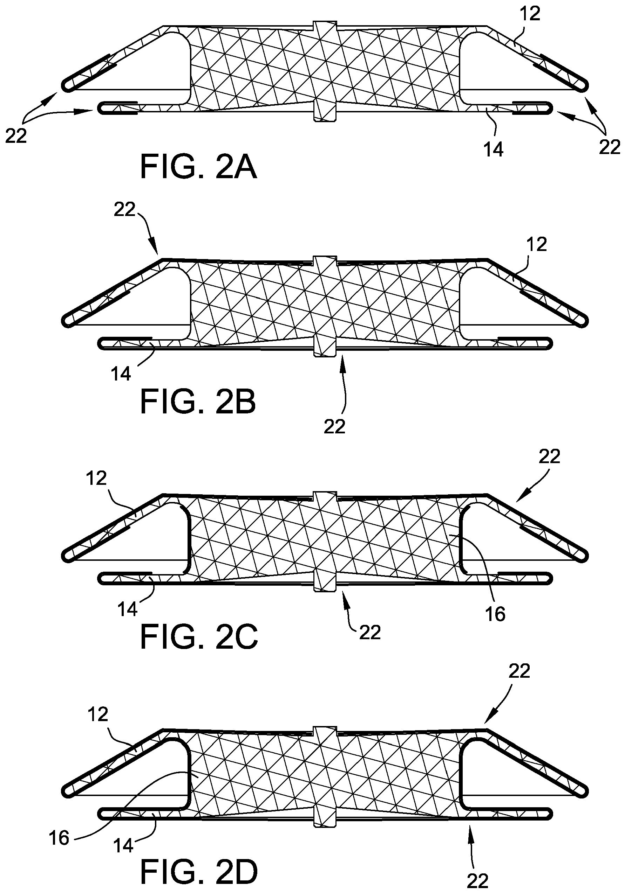

FIGs. 2A-D, 3A-B, and 4A-E illustrate exemplary embodiments of device coatings, including fabric coatings that cover at least portions of proximal and distal discs, encapsulation coatings that cover at least portions of proximal and distal discs, and coatings covering at least a portion of the waist member by either fabric coating and/or encapsulation coating, respectively.

### a. Fabric Coating

In some embodiments of the present disclosure, the medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site. The medical device also includes a fabric coating covering at least one of the proximal disc portion and the distal disc portion. In some embodiments, the friction of the medical device on the cardiac tissue is eliminated or reduced by decreasing a surface roughness of the medical device.

By affixing fabric to the device (multiple configurations are possible: such as fully encapsulating an outer or inner surface of the device, fully encapsulating an outer or inner surface of at least one disc, or partially encapsulating at least one disc (e.g., around the disc edges or flat disc sections, see FIGs. 2A-D) of the device, the surface roughness of the exposed braid is significantly decreased by more evenly applying pressure to the cardiac tissue and lowering the coefficient of friction (depending on material).

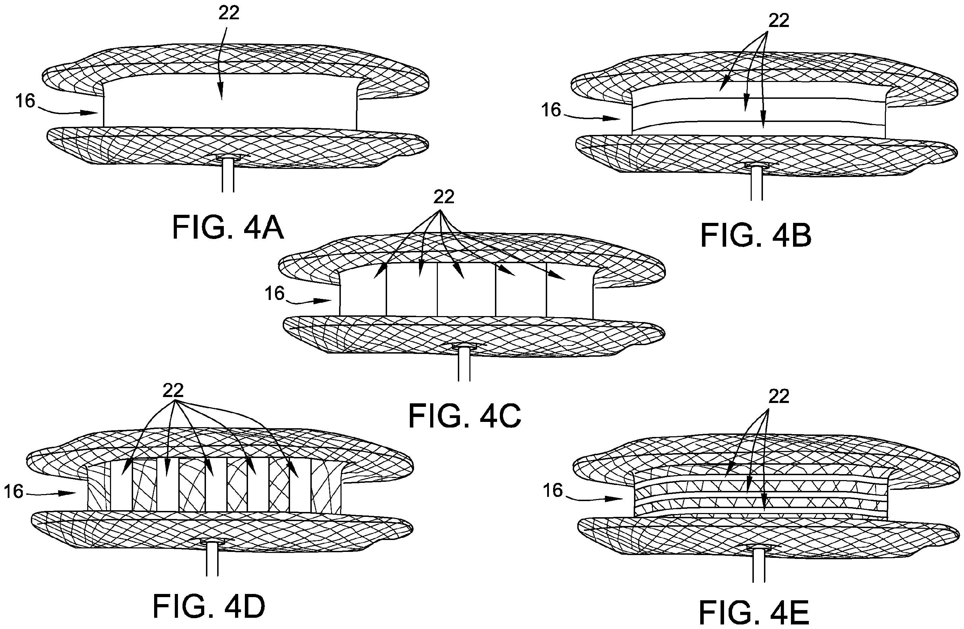

By affixing fabric to areas of the waist member (multiple configurations are possible (see FIGs. 4A-E), the radial force of waist is increased. In some embodiments, fabric may be affixed to cover the entire waist member (e.g., covering the entire circumference of the waist member either by a single continuous covering or by multiple sections that together provides continuous coverage.

In some embodiments, the fabric is affixed through sewing, adhering, laminating, electrospinning (see below) or another method. With fabric wrapped around the disc edges, the ridges/roughness formed by large openings in the braid is significantly smoothed, decreasing the abrasiveness of the medical device. Depending on the material chosen (polyester, PFTE, ePTFE, etc.), the fabric's interaction with tissue can be adjusted to best accommodate the device needs for permeability and tissue ingrowth.

In some embodiments, the fabric coating is affixed to at least one of the surface of the proximal disc portion and the surface of the distal disc portion. In some embodiments, the fabric fully encapsulates at least one of the proximal disc portion and the distal disc portion. In some embodiments, the fabric partially encapsulates at least one of the proximal disc portion and the distal disc portion.

In some embodiments, the fabric coating 22 is affixed to the disc edges of discs 12 and/or 14. As shown in FIG. 2A, the fabric 22 is attached only to the edge of the discs 12, 14, which provides a buffer layer between tissue and the braid.

In some embodiments, the fabric coating 22 completely covers the discs 12 and/or 14, wherein the fabric 22 encompasses each disc 12, 14 completely and wraps around the edges (see, e.g., FIG. 2B). This arrangement allows the removal of the inner fabric and provides a buffer layer between the tissue and the braid.

In some embodiments, the fabric coating 22 is on both the discs 12, 14 and the waist 16, but is separated, as shown in FIG. 2C. This further cushions the tissue/braid interaction. Further, as the pieces are separated, this arrangement minimizes the need for the fabric to stretch with the braid as it elongates.

In some embodiments, the fabric coating 22 covers the entire occluder (see, e.g., FIG. 2D). In some embodiments, the fabric is all one piece (similar to a sock covering the entire occluder).

It is understood that the pictures shown in FIGs. 2A, 2B, 2C and 2D are exemplary cross-sections of the occluder shape, and in some embodiments, the fabric 22 wraps all the way around the diameter of the occluder.

In some embodiments, fabric 22 covers all or part of the waist member 16, as shown in FIGs. 4A-4E. In embodiments when fabric 22 forms a continuous cover around the circumference of waist member 16 as well as a continuous cover over an axial length of waist member 16, the fabric may be all one piece/section (FIG. 4A) or several sections applied/attached adjacent to one another (FIGs. 4B and 4C) to form the continuous covering. In other embodiments, waist member 16 is partially covered by fabric 22 in one or more sections (FIGs. 4D and 4E). Size and shape of sections of fabric 22 may vary by according to the embodiment. For example, length of fabric 22 sections may vary depending on the stiffness of the material used (i.e. how much resistance it will add to radial compression/elongation at the waist) and the desired stiffness of the waist. Further, application of circumferential sections of fabric 22 provides an additional way to adjust and optimize radial force at the waist of the device.

In some embodiments, the fabric coating comprises at least one of polyester (knit, woven or non-woven), electrospun thermoplastic polyurethane (TPU), polytetrafluoroethylene (PTFE) and expanded polytetrafluoroethylene (ePTFE).

In some embodiments, the fabric is attached via sewing (to the braid itself), adhered (with TPU or other similar material), laminated (melted directly to the braid) or electrospun onto the braid.

### b. Braid Encapsulation Coating

In some embodiments of the present disclosure, the medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer is encapsulated with a polymer coating. In some embodiments, the friction of the medical device on the cardiac tissue is eliminated or reduced by decreasing a surface roughness of the medical device.

By coating the braid through dipping in a liquid polymer, then curing it, the medical device reduces or eliminates the need for fabric while benefiting from the same result in reduction of friction by more evenly applying pressure to the cardiac tissue and lowering the coefficient of friction (depending on material). In particular, by coating the braid with a liquid polymer, whether internally, externally or a hybrid of both, the medical device reduces or eliminates the need for fabric while benefiting from the same result in reduction of friction by more evenly applying pressure to the cardiac tissue and lowering the coefficient of friction (depending on material). In some embodiments, coating is done via dipping, spray-coating, electrospinning (see below) or another method. The benefits of braid encapsulation include the removal of a need for inner fabric or sutures and a reduction in the coefficient of friction and abrasiveness near the edges due to a covering of the braid wires with a material that fully encloses the medical device.

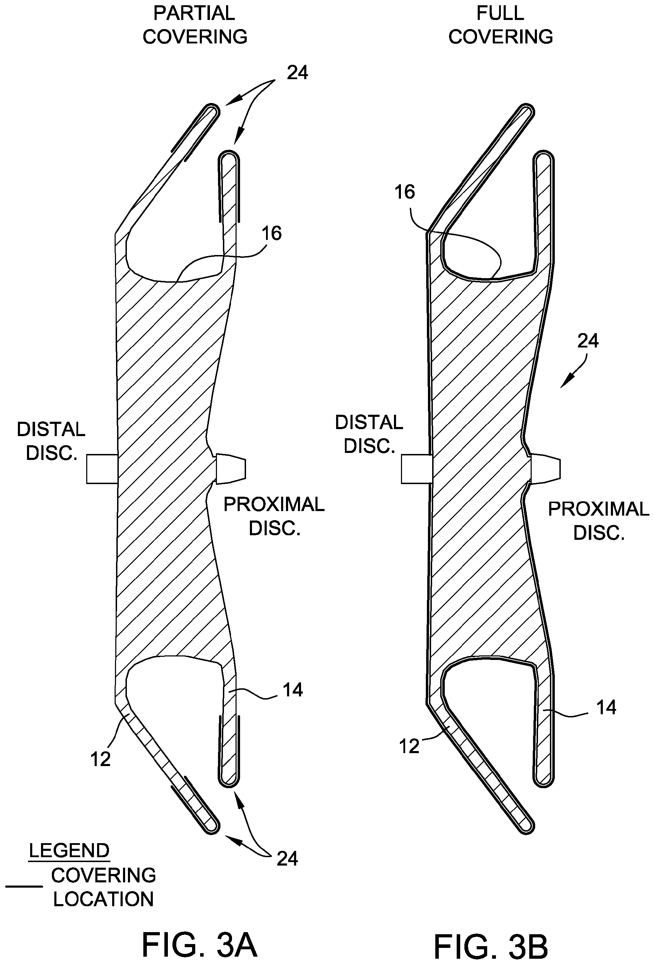

In some embodiments, the at least one braided layer comprises an internal coating, an external coating, or both. In some embodiments, the at least one braided layer is coated through at least one of dipping, spray-coating and electrospinning. In some embodiments, the polymer coating fully encapsulates the at least one braided layer. In some embodiments, the polymer coating partially encapsulates the at least one braided layer. In some embodiments, specific braid encapsulation coatings are urethane or silicone-based, via a dip or spray application. In some embodiments, to promote ingrowth, the coatings are perforated with small holes via a laser. In some embodiments, the coatings are applied over the entire device, or on the edge of the disc (e.g., left atrial disc 12 and/or right atrial disc 14) only to act as a protective barrier or bumper. FIGs. 3A (partial covering) and 3B (full covering) show where a covering 24 is applied, although the covering 24 could be applied to more or less of the device than shown in the exemplary embodiment. Similarly to fabric coating 22 embodiments shown in FIGs. 4A-4E, encapsulation coating/covering 24 may be applied to waist member 16 of the device to increase radial force. For example, covering 24 may be applied as a single section to form a continuous covering around both the axial length and circumference of waist member 16 (similar to FIG. 4A), covering 24 may be applied as several sections adjacent to one another to form a continuous covering (similar to FIGs. 4B and 4C), or covering 24 may be applied in one or more sections to partially cover waist member 16 (similar to FIGs. 4D and 4E). Size and shape of sections of covering 24 may vary by according to the embodiment. For example, length of covering 24 sections may vary depending on the stiffness of the material used (i.e. how much resistance it will add to radial compression/elongation at the waist) and the desired stiffness of the waist member 16. Further, application of circumferential sections of covering 24 provides an additional way to adjust and optimize radial force at the waist of the device. In some embodiments, a combination of one or more fabric 22 sections and one or more covering 24 sections may be utilized to provide the desired stiffness of the waist member 16.

In some embodiments, the polymer coating comprises at least one of polyurethane or silicone. In some embodiments, the preferred coating comprises polyurethane.

In some embodiments, the braided layer comprises a material selected from the group consisting of stainless steel, nickel-based, cobalt-based, nickel-titanium, shape memory and super-elastic materials. One class of materials which meets these qualifications is the class of shape memory alloys. One particularly preferred shape memory alloy for use in the present disclosure is Nitinol. NiTi alloys are also very elastic-they are said to be "superelastic" or "pseudoelastic". This elasticity may allow the device to return to a preset expanded configuration for deployment following passage in a distorted form through a delivery catheter. In some embodiments, the braided layer comprises at least one of nylon, polypropylene, polyvinyl alcohol (PVA), polyester, and combinations thereof.

It is also understood that the device may comprise various materials other than Nitinol that have elastic properties, such as spring stainless steel, trade named alloys such as Elgiloy®, or Hastalloy, Phynox®, MP35N, CoCrMo alloys or a mixture of metal and polymer fibers. Polymer fibers may include monofilaments or multifilament yarns ranging from about 10-400 denier. Individual filaments may range from about 0.25 to 10 denier. Polymers may be composed of PET (Dacron™), polyester, polypropylene, polyethylene, HDPE, polyurethane, silicone, PTFE, polyolefins and ePTFE. The metal and plastic fibers may be combined in the same layer, or the tubular layers may be constructed in such a manner that each layer is made from a different material. The polymer layer may be a multifilament braided layer or may be composed of at least one filament or yarn wound about a mandrel with a pitch and diameter similar to other adjacent layers and may be positioned about or inside another adjacent layer or between adjacent layers. Depending on the individual material selected, the wire strand diameter, number of wire strands and pitch may be altered to achieve the desired properties of the device. Moreover, other suitable materials include those that are compatible with magnetic resonance imaging (MRI), as some materials may cause heat or torque resulting from performing MRI, and some materials may distort the MRI image. Thus, metallic and/or non-metallic materials that reduce or eliminate these potential problems resulting from using MRI may be employed.

### c. Braid Parylene Coating

In some embodiments of the present disclosure, the medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, and wherein a parylene coating covers at least a portion of the at least one braided layer. In some embodiments, the friction of the medical device on the cardiac tissue is eliminated or reduced by decreasing a surface roughness of the medical device.

In some embodiments, a thin layer of parylene deposited on the formed medical device reduces the coefficient of friction of the braid (or fabric) that contacts cardiac tissue. In particular, in some embodiments the parylene coating deposition results in an extremely thin layer of lubricious polymer on all of the exposed surfaces of the medical device. This improves the ease of the device's travel down the delivery sheath, may result in the resolution of the cobra formation, and decreases the coefficient of friction of the wires. It is also understood that in some embodiments the parylene braid coating is paired with other solutions to lower the coefficient of friction in other scenarios without contributing to device profile.

In some embodiments, an exposed portion of the proximal disc portion and an exposed portion of the distal disc portion comprise a parylene coating. In some embodiments, the at least one braided layer is coated through at least one of dipping, spray-coating and electrospinning. In some embodiments, the parylene coating fully encapsulates the at least one braided layer.

In some embodiments, the process of parylene coating is a standard process known in the industry. The materials to be coated (the assembled occluder, before fabric attachment) are loaded into a coating chamber where the parylene polymer is atomized and deposited on the surface. In some embodiments, the coating thickness is very thin, often ranging from about 0.1 to about 50 microns. If needed, parts of the occluder that shouldn't be coated are masked (such as the end screw).

A parylene coating lowers the coefficient of friction on the braid wire and therefore lowers the potential friction on the tissue, thereby leading to less damage.

### d. Polymer Electrospinning Onto Braid

In some embodiments, the medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer comprises a polymeric fabric coating located on an outside surface of the braided layer, wherein the polymeric fabric coating is deposited on the outside surface of the braided layer through an electrospinning process. In some embodiments, the friction of the medical device on the cardiac tissue is eliminated or reduced by decreasing a surface roughness of the medical device.

In some embodiments, through the use of an electrospinning process, a thin layer of fabric is deposited on the outside surface of the braid, and more evenly applies pressure to cardiac tissue and lowers the coefficient of friction (depending on material) of the device. The electrospinning process involves the spinning of the device (e.g., occluder) and dispensing a liquid polymer into the electrical field within which the device is contained. This results in a non-woven fabric conformed to the shape of the device. During spinning, the device is stretched or non-stretched depending on the need. The benefits of this embodiment include that the fabric does not need to be sewn on, the fabric could cover the whole device or part, the thickness is tailored to the need, and like the other fabric solutions, the coefficient of friction is reduced along with the device abrasiveness against the tissue.

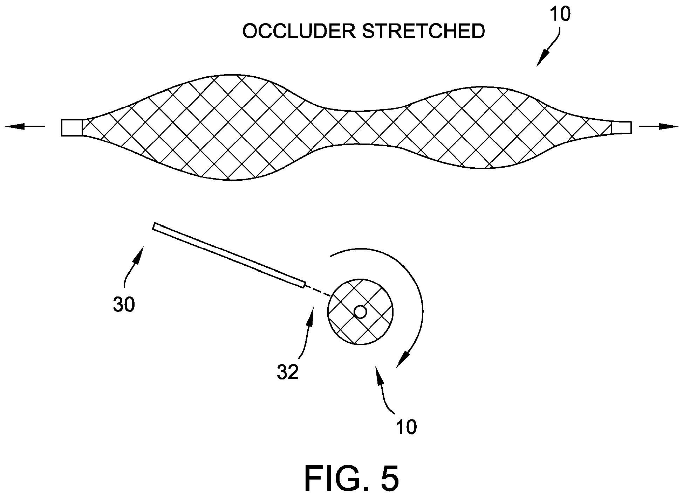

FIG. 5 depicts an exemplary embodiment of a device 10 with an electrospun coating applied, using a needle 30 to apply the polymer fabric 32 to the device 10.

In some embodiments, the fabric coating has a thickness of from about 0.0005 inches to about 0.005 inches. In some embodiments, the fabric coating comprises a non-woven fabric. In some embodiments, the fabric coating conforms to the shape of the medical device.

In some embodiments, the medical device is stretched during the electrospinning. In some embodiments, the medical device is not stretched during the electrospinning. In some embodiments, the electrospinning includes spinning the medical device and dispensing a liquid polymer into an electrical field within which the medical device is contained.

The electrospinning process is used to apply the coating to the braid, which creates a porous structure that is dense enough to occlude the defect. The pores promote tissue ingrowth. In some embodiments, a urethane-based polymer is used for this application; however, several other polymers can also be electrospun, including, but not limited to, nylon, polypropylene, PVA, PTFE and polyester. In some embodiments, the materials used in biomedical electrospinning include, but are not limited to, polyglycolic acid (PGA), PEG, PU, poly(lactic acid)(PLA), poly(ethylene-co-vinyl acetate) (PEVA), polycaprolactone (PCL), poly-1-lactide (PLLA), and polyvinyl alcohol (PVA), poly ε-caprolactone (PCL), salicylic acid (SA), polyethylene glycol-poly(lactic acid), poly(propylene glycol) (PPG),poly-L-lactide-co-ε-caprolactose (PLLA-CL-); and, poly-lactide-co-glycolid (PLGA).

### e. Lubricious Ceramic Coating

In some embodiments, the medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, wherein the at least one braided layer comprises a ceramic coating on an outside surface of the braided layer. In some embodiments, the friction of the medical device on the cardiac tissue is eliminated or reduced by decreasing a surface roughness of the medical device.

In other embodiments, the coating is a platinum coating, which reduces friction, increases radio-opacity, increases corrosion resistance and reduces nickel leaching.

Through the use of vacuum-based processes like physical vapor deposition (PVD), chemical vapor deposition (CVD), in some embodiments the surface is coated by materials like diamond-like carbon and titanium nitride, and a thin layer of coating is deposited on the outside surface of the braid, which lowers the coefficient of friction of the medical device. In particular, deposition processes, like PVD, CVD involve controlled deposition of thin layers under vacuum conditions. The technology involves deposition of coating by using single material or mixture of materials like gases (methane, argon or titanium and nitrogen) to form thin layers of lubricious coating. One of the benefits of these embodiments is that the device can be coated after forming, just covering the wires without affecting the open cells between the braid wires. The coefficient of friction is reduced along with the device abrasiveness against the tissue.

In some embodiments, the ceramic coating comprises at least one of diamond-like carbon, titanium nitride, titanium carboNitride (TiCN) zirconium nitride (ZrN), titanium niobium nitride (TiNbN), chromium nitride (CrN), and titanium oxide.

In some embodiments, the coating has a thickness of from about 10 nm to about 2µm.

In some embodiments, the coating is applied through physical vapor deposition (PVD) or chemical vapor deposition (CVD). In some embodiments, the medical device is coated after forming.

### Increasing Medical Device Compliance

In some embodiments of the present disclosure, the medical devices disclosed herein reduce and/or eliminate erosion of cardiac tissue while maintaining the fundamental function and effectiveness of an occluder (such as, for example, an Amplatzer™ Septal Occluder (ASO)). In some embodiments, the medical devices achieve this objective by increasing the medical device compliance to cardiac structures and movement.

By increasing medical device (e.g., occluder) compliance, the device moves more freely with device tissue, thus reducing normal forces between the disc and the tissue as well as device movement relative to the tissue. Through reduction of normal forces and device-tissue movement, the frictional interaction between the device and tissue decreases and thus also does the likelihood of erosion.

### a. Braid Pattern

Tissue erosion due to the friction between the occluder medical device and the tissue after atrial septal defect closure is a risk for braided occluders. The friction force may be lowered by making the occluder softer (i.e., more compliant); however, a softer device may be more prone to bulging into the atrium(s) as forces are applied to the waist member and disc portions of the occluder. Heart block is a known issue with VSD occluders, and radial force of the occluder waist member may be a contributing factor. Waist member softness (or stiffness) is affected by the braid wire diameter, number of braid wires, braid pattern/density of the braided layer covering the waist member, the shape of the waist member, and the adjacent braid that covers the proximal and distal discs.

Braided occluders are typically made with a braid diameter that closely matches a diameter of a largest portion of the occluder. In exemplary embodiments, braid diameter is defined by a maximum expanded braid diameter and is a function of a diameter of the braid mandrel on which the braid is formed as well as a pic rate (i.e., pics per inch, or PPI) of the braid. In braided materials, PPI describes the number of braid wire crossings per inch of material, in which a pic (sometimes also referred to as a 'pick') is a single crossing of braid wires. Braid wire size (i.e., wire diameter) is dependent on multiple factors, including the size of the device, the forces required to secure the device in the anatomy, the number of braid wires (e.g., PPI), the purpose of the braid layer (occlusion vs. embolization resistance), the location of the defect, etc. In some embodiments, suitable wire sizes for braids used to form braided occluders of the present disclosure are in the range of about 0.0015" diameter to about 0.008" diameter wire.

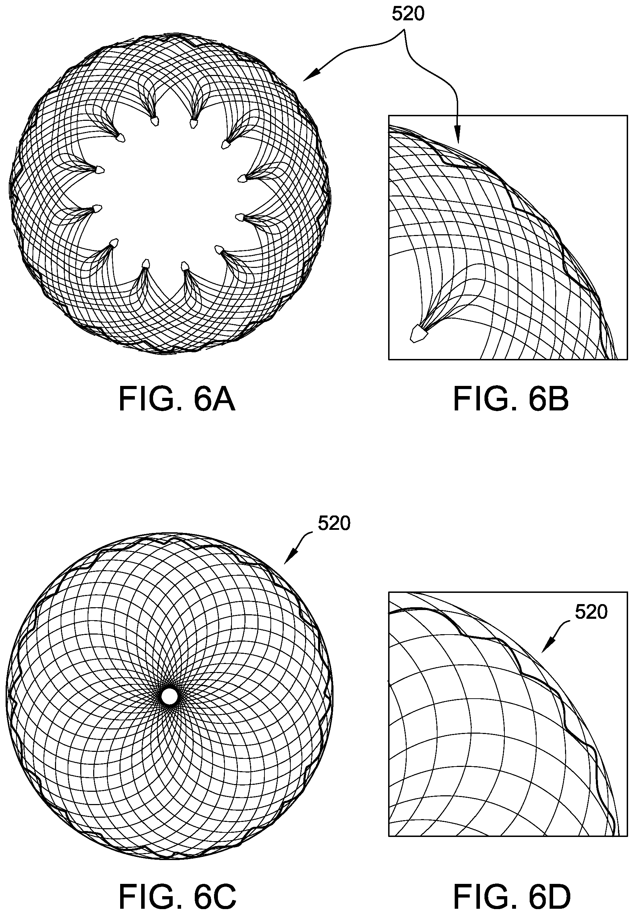

By way of example, in an occluder having a distal disc portion with a diameter of 40mm, the braid forming a braided layer on the occluder is typically braided on a 40 mm mandrel. Consequently, at a suitably high pic rate, the maximum expanded diameter remains equal to the diameter of the mandrel at approximately 40 mm (e.g., is not much larger than the 40mm distal disc portion, see, e.g., FIGs. 6A and 6B). The mandrel, along with the pic rate of the braided layer, defines the helix length of the braided layer along the length of the formed device. As used herein, "longer" and "shorter" helix lengths refer to relative axial distance covered by a single revolution of a helical wire. By way of example, a wire having a "longer" helix length will extend a greater axial distance per helical revolution and have a greater helical pitch than a wire having a "shorter" helix length. It will therefore be understood that a longer helix length wire will require less wire per unit of axial length than a shorter helix length wire.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The medical device further comprises at least one braided layer, wherein the at least one braided layer has a braid diameter greater than a diameter of a largest portion of the medical device. In some embodiments, the medical device compliance on cardiac tissue is increased while the friction force between the medical device and the tissue is lowered by increasing a braid diameter and helix length of the at least one braided layer, consequently increasing a softness and/or reducing a stiffness (e.g., radial stiffness) of the medical device.

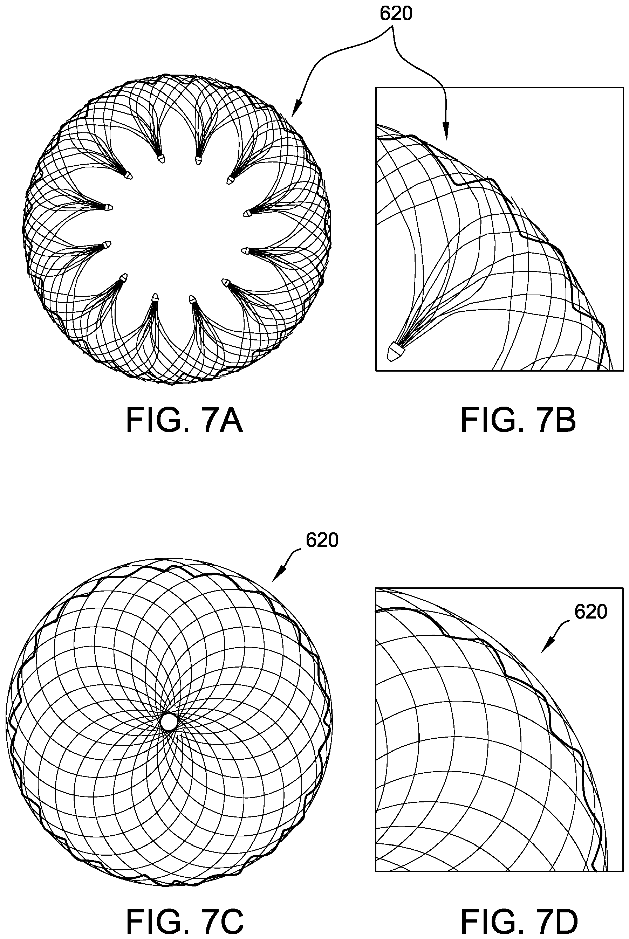

FIGs. 6A-D show a 26 mm occluder having a 40 mm diameter distal disc formed with a braided layer 520 having a 40 mm braid diameter (e.g., braided on a 40 mm mandrel) such that the braid diameter is equal to the largest disc diameter. FIGs. 7A-D show a 26 mm occluder having a 40 mm diameter distal disc formed with a braided layer 620 having a 56 mm diameter such that the braid diameter is greater than the largest disc diameter. The braided layer 620 having a 56 mm diameter may be formed, for example, by braiding the braided layer on a 56 mm mandrel at a suitably high pic rate such that the maximum expanded braid diameter is 56 mm, or alternatively braiding the braided layer on a mandrel having a diameter less than 56 mm and at a relatively lower pic rate, such that the maximum expanded braid diameter is 56 mm. Particularly from the enlarged views of FIGs. 6B, 6D, 7B, and 7D, embodiments having a braid diameter that is greater than the largest disc diameter (FIGs. 7A-D) results in a lower PPI when compared to embodiments having a braid diameter that is equal to the largest disc diameter (FIGs. 6A-D). Accordingly, by utilizing a larger braid diameter and/or by lowering PPI to form the braided layer (for example, using a 50+ mm diameter braid to form a 26 mm occluder having a largest-disc diameter of 40 mm) the helix length increases on the formed device. A longer helical length generally reduces radial stiffness and bulging of the deployed device. However, a longer helical length may result in increased axial stiffness, increased force required to collapse the device, increased strain on the material of the device during loading and fatigue, and could therefore reduce the fatigue life of the implanted/deployed device. As helix length is defined by mandrel size and pic rate (i.e., braid diameter), optimal braid diameter varies from embodiment to embodiment. Optimal braid diameter is a balance of the above-mentioned factors (radial stiffness, device bulging, axial stiffness, device collapsibility, material strain, and device fatigue life), and may include different braid diameters and/or PPIs across the formed device.

Notably, although FIGs. 6A, 6B, 7A, and 7B illustrate the various braid diameter features disclosed herein as implemented in a "halo"-type device, where the discs have a central opening defined therein, it should be readily understood that the braid diameter features disclosed herein may be implemented in any type of medical device, including other occluders with continuous discs as exemplified in FIGs. 6C, 6D, 7C, and 7D (such as, for example, an Amplatzer™ occluder device).

In exemplary embodiments, an increased helix length makes the expanded configuration length of the device shorter, which may be beneficial for attaching fabrics or materials with less elongation to the braid/braided layer. Attaching fabric to two points along the length of the braid may be beneficial for fabric coverings that address erosion, fabric coverings on occlusion devices (such as Abbott's Amulet™ devices) to promote tissue growth near the stabilizing members, fabric inserts in braided grafts, a soft fabric waist member in a VSD or ASD device to lower the radial force, polymer adhesion via dipping/spraying/electrospinning to reduce the friction of the device and promote tissue ingrowth, polymer adhesion to obstruct blood flow without the need for sewn patches, etc.

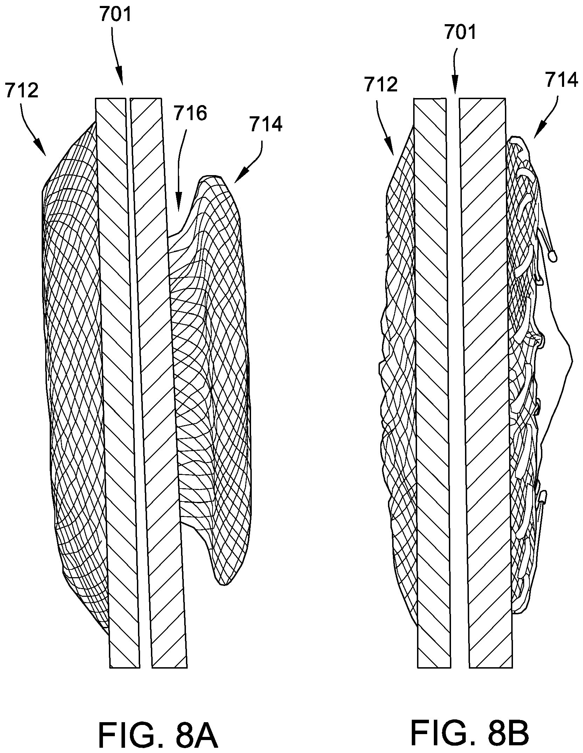

Additionally, the longer helix length changes the mechanical properties of the device because of the path the braid wires take as they wrap around the edges of the disc and waist of the device. The more direct wire path around the device allows it to hold its shape better when forces are applied to the disc and waist, which means bulging can be minimized when a smaller wire diameter is used (see FIGs. 8A and 8B for a bulging comparison between a standard braid diameter and a larger braid diameter, respectively), thereby permitting the use of braids with smaller diameter wires. The smaller wire diameter lowers the forces that the device imparts on the tissue and, therefore, the risk of erosion resulting from tissue abrasion. In some embodiments, suitable wire sizes for braids used to form braided occluders of the present disclosure are in the range of about 0.0015" diameter to about 0.008" diameter wire, or more suitably from about 0.0035" diameter wire up to about 0.006" diameter wire. In one particular embodiment, a 14 mm braided occluder device is formed with a braided wire having a wire diameter of about 0.0035". In another particular embodiment, a 40 mm braided occluder device is formed with a braided wire having a wire diameter of about 0.0055".

As shown in FIG. 8A, an occluder device having a standard braid diameter is deployed across septum 701, resulting in increased bulging of distal disc 712 and proximal disc 714. In the expanded, deployed configuration of FIG. 8A, waist member 716 also exhibits bulging. FIG. 8B shows an occluder device having a larger braid diameter deployed across septum 701. Distal disc 712 and proximal disc 714 of FIG. 8B exhibit decreased bulging as compared to FIG. 8A, due to the larger braid diameter of the device shown in FIG. 8B. Further, waist member bulging is not exhibited by the device shown in FIG. 8B.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein a first portion of the at least one braided layer covers the waist member and has a different braid pattern than a second portion of the at least one braided layer covering at least one of the proximal disc portion and the distal disc portion. In some embodiments, the medical device compliance on cardiac tissue is increased while the friction force between the medical device and the tissue is lowered by changing a braid pattern of the waist member to have fewer pics per inch than the braid pattern covering the disc portions, thereby forming a softer waist member of the medical device. In some embodiments, radial force near the waist is maintained (or increased) and disc bulging is limited by keeping the PPI near the waist and inside portions of each disc relatively high, and further by reducing the PPI around the disc edge(s). Depending on the embodiment, increasing the PPI in the waist of the device effectively adds more braid wire to the waist, which allows the waist to elongate more to accommodate thicker septums, and provides a stronger radial force to better fill the defect. Further, more wire in the waist enables elongation and increased flex in the waist. Additionally, the increased amount of wire (reduced helix length) in the waist allows the discs to accommodate a wider range of septal thicknesses without significantly changing the diameter of the waist and affecting the adjacent braid on the underside of the discs; this helps the device maintain its shape, which reduces bulging after deployment. The higher PPI also provides more flexibility to accommodate forces generated by the pressures across the atrium, motion of the atrial septum, and other forces acting on the device. Examples of other forces acting on the device include, but are not limited to, external blunt trauma, chest compressions during CPR, and aortic root movement during the cardiac cycle.

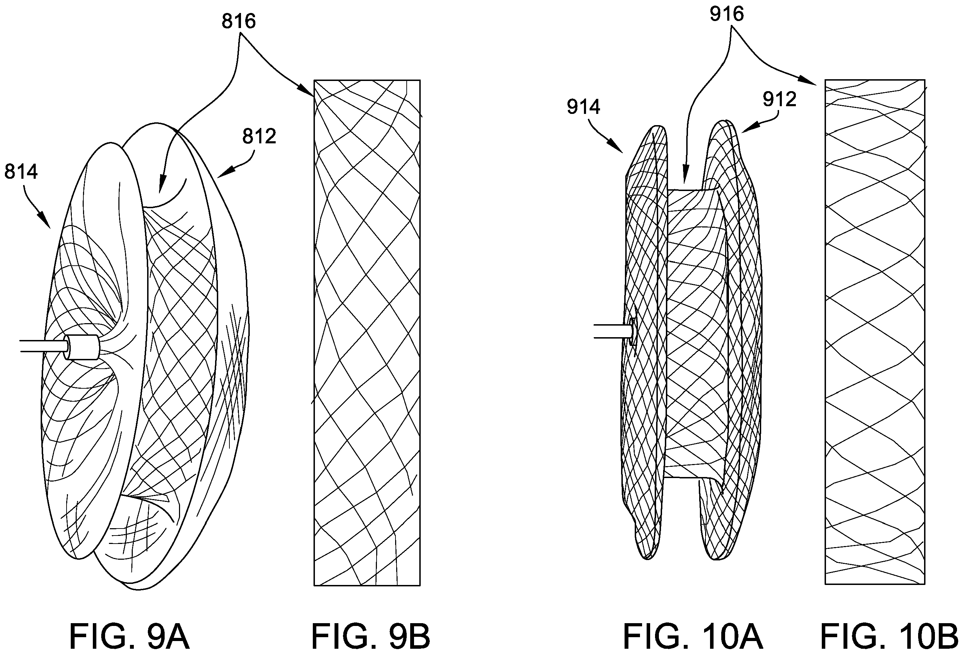

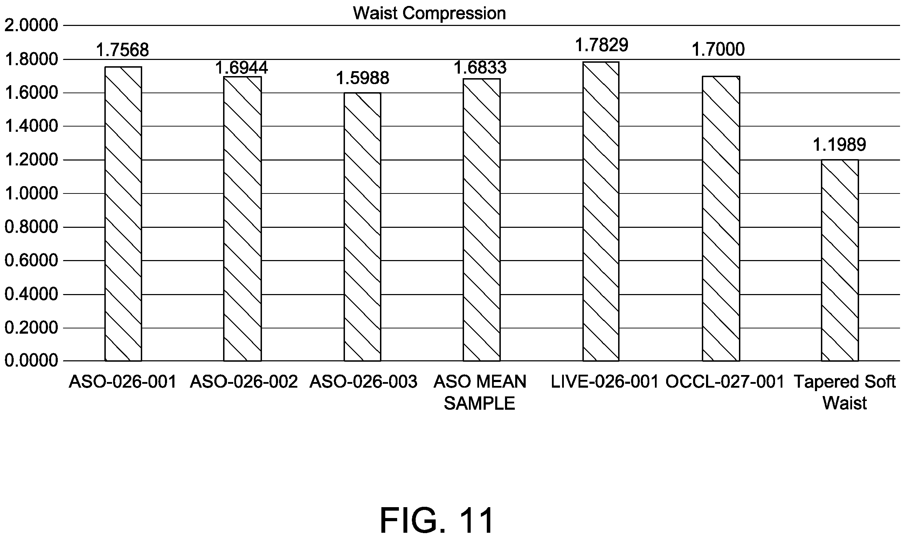

The braid pattern covering the waist member of the device can be affected by modifying the braid diameter, the PPI, or by simply modifying the mandrel that defines the shape of the waist member (see FIGs. 9A-B and 10A-B for a standard ASO waist and a tapered prototype with lower PPI waist braid pattern, respectively). FIGs. 9A-B show distal disc 812, proximal disc 814, and waist member 816. FIGs. 10A-B show distal disc 912, proximal disc 914, and waist member 916. Embodiments having a lower PPI as shown in the enlarged view of FIG. 10B result in a generally softer waist member 916 when compared with embodiments having a higher PPI as shown in the enlarged view of FIG. 9B (which have a generally stiffer waist member 816). Consequently, an occluder having a softer waist member (lower waist PPI and longer helix length) exhibits a lower waist compression when compared with an occluder having a stiffer waist member (higher waist PPI and shorter helix length). Further, the longer helix length within the waist of the device lowers the waist compression/radial strength (see FIG. 11), which may reduce the risk of tissue erosion by allowing the waist member to give when a force is applied to the disc. A softer waist may also be beneficial for VSD devices that are known to have a high rate of heart block. Conversely, there may be designs that benefit from a stiffer waist to help center the device within the defect, in which case increasing the PPI at the waist member is necessary.

In one embodiment, the present disclosure is directed to a medical device for treating a target site. The medical device comprises a tubular member comprising a proximal disc portion at a proximal end, a distal disc portion at a distal end, and a waist member extending between the proximal disc portion and the distal disc portion. The tubular member has an expanded configuration when deployed at the target site and a reduced configuration for delivery to the target site. The tubular member comprises at least one braided layer, wherein a pics per inch of the at least one braided layer changes along a length of the medical device. In some embodiments, the medical device compliance on cardiac tissue is increased, and friction force between the medical device and the tissue is lowered.

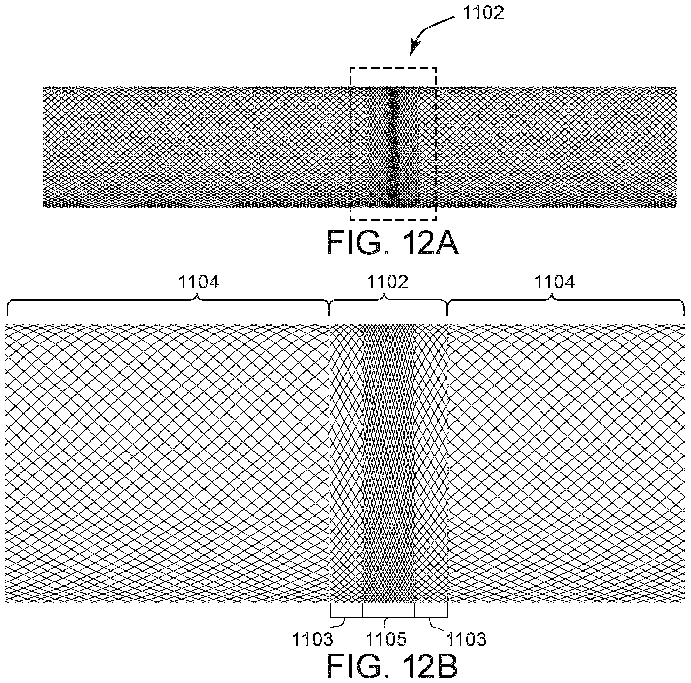

Lastly, the mechanical properties of the device, and elongation in specific sections, can be further tailored by changing the pic rate (i.e., PPI) along the length of the braid. FIG. 12A illustrates a braid pattern on a mandrel where the braid pattern has a changing pic rate across a length of the braid. Specifically, the pic rate at a center portion 1102 (e.g., a waist portion) of the braid is higher than a pic rate at outer portions of the braid pattern located distally and proximally to the center/waist portion 1102 (e.g., distal and proximal discs). FIG. 12B is an enlarged view of the braid pattern shown in FIG. 12A, in which the pic rate at the center/waist portion 1102 includes transitional portions 1103 and central portion 1105 having higher pic rates than a pic rate at disc portions 1104. Specifically, central portion 1105 has a higher pic rate than transitional portions 1103. Lower and higher pic rates can be positioned within the discs, near the disc edges, and at the waist to affect the waist stiffness, clamping force, disc stiffness, and device bulging upon implant. In some embodiments, optimal stiffness at the waist and discs is dependent on exact locations of the PPI changes across the device. The amount that these properties can be tuned is limited by the amount the PPI can change across the braid by modifying the PPI with braider settings.

In some embodiments, the waist braid configuration shown in FIGs. 12A-B may be achieved using chase wires. That is, additional wires (or chase wires) of the same or larger diameter of wires used in the braid may be braided in conjunction with any number of wires in the braid and then removed from (e.g., cut out of) all but the device waist. Chase wire ends may need to be secured to adjacent wires (such as those forming the waist and/or disc portions) or formed/directed inward towards the center of the device to prevent any traumatic wire ends during delivery, deployment, and defect occlusion.

In other embodiments, the waist braid configuration shown in FIGs. 12A and 12B may be achieved using wire forms. Formed wires of any shape, size or number may be circumferentially interwoven into the braid only at the waist of the device. Alternatively, the additional wires may be formed separately and attached to the waist of the device by suturing or laminating the wire form to the device waist.

In some embodiments, a braided occluder is made with a braid pattern that transitions to a higher PPI near the waist and a lower PPI near the discs. For instance, a first section is braided at a low PPI (e.g., approximately 15 PPI) so that the braid can expand to the largest disc diameter, which is followed by braiding at a relatively high PPI (e.g., approximately 40 PPI) for the center/waist section (e.g., approximately .25" long), and again at a lower PPI (e.g., approximately 15 PPI) for a last section for the second disc. This higher waist PPI can be achieved by changing the PPI and braiding on a smaller mandrel than is typically utilized for a given sized device, e.g., the mandrel diameter approximates the waist diameter instead of the largest disc diameter. Alternatively, the higher waist PPI can be achieved by braiding on a tapered mandrel, or using a combination of braiding on a smaller mandrel and braiding on a tapered mandrel. In some embodiments, the PPI (at a specific diameter) can also be modified by braiding on a mandrel that changes diameter across the length of the mandrel (e.g., a collapsible mandrel to allow removal of the finished braid).

In embodiments where the central/waist section PPI is higher than the outer/disc section PPI, bulging can be reduced. Further, radial force/hoop strength is increased in the waist to better fill out the defect, while the amount of braid in the waist area and/or the underside of disc area is also increased, allowing it to extend further to accommodate a thicker septum. Further, when the device is better able to fill out the defect, occlusion is improved and the risk of residual leaking is reduced. While not being bound by any particular theory, shortened helix pitch reduces strain on the braid wire near the waist because the wire takes a more gradual path from waist-to-disc, as well as the braid wire near the disc edge because it is more likely to maintain its formed shape throughout the cardiac cycle.

FIGs. 13A-F, 14A-B, and 15A-B illustrate comparisons between braided occluders having transitioned (changing pic rate) and standard (generally consistent pic rate) braid patterns in a deployed state. FIGs. 13A and 13B compare the proximal discs 1214 by showing a transitioned braided occluder with a changing pic rate in thick septum 1201, and a standard braided occluder with a generally consistent pic rate in thick septum 1201, respectively. Distal discs 1212 are also shown in FIGs. 13A and 13B. FIGs. 13C and 13D, and, similarly, FIGs. 13E and 13F, compare distal discs 1212 by showing a transitioned braided occluder with changing pic rate in thick septum 1201, and a standard braided occluder with a generally consistent pic rate in thick septum 1201, respectively. When deployed at a given septal defect target site having thick septum 1201 (as shown in FIGs. 13A-F), the transitioned braided occluders (FIGs. 13A, 13C, and 13E) exhibit less bulging than the comparable standard braided occluders (FIGs. 13B, 13D, and 13F).

Notably, although one or more of FIGs. 13A-13D may illustrate the varying pic rate features disclosed herein as implemented in a "halo"-type device, where the discs have a central opening defined therein, it should be readily understood that the varying pic rate features disclosed herein may be implemented in any type of medical device, including other occluders with continuous discs as exemplified in FIGs. 13E and 13F (such as, for example, an Amplatzer™ occluder device).

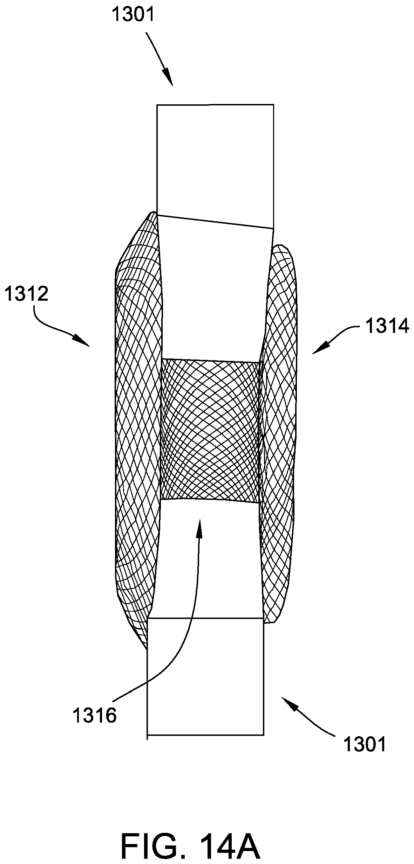

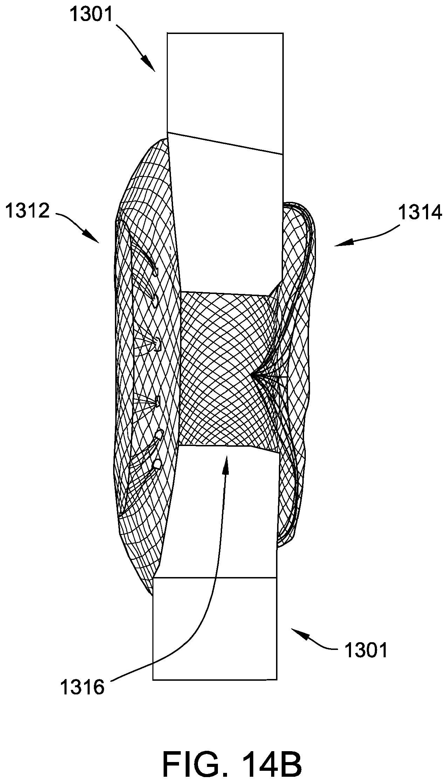

FIGs. 14A and 14B show a septum 1301 cutaway of a transitioned braided occluder with a changing pic rate and a standard braided occluder with a generally consistent pic rate, respectively. FIGs. 14A and 13B show distal disc 1312, waist member 1316, and proximal disc 1314 as deployed in/across septum 1301. When deployed at a given septal defect target site having septum 1301 (as shown in FIGs. 14A and 14B), the transitioned braided occluder (FIGs. 14A) exhibits less puckering/buckling than the comparable standard braided occluders (FIGs. 14B), thereby providing improved defect coverage and occlusion.

FIGs. 15A and 15B show a top view (i.e., proximal end-facing view showing proximal disc coverage of the defect) of a transitioned braided occluder with a changing pic rate (FIG. 15A), and a top view (i.e., proximal end-facing view showing proximal disc coverage of the defect) of a standard braided occluder with a generally consistent pic rate (FIG. 15B). Both FIGs. 15A and 15B show a proximal end-facing view of the occluder when deployed at a target site (e.g., at a septal defect 1402), having a thick tapered septum. FIG. 15A shows a distance 1404 from defect 1402 to an outer edge of the proximal disc. FIG. 15B shows a distance 1406 from defect 1402 to an outer edge of the proximal disc. FIG. 15C shows a side-by-side comparison of the transitioned (right) versus standard (left) braid patterns illustrated in FIG. 15A and FIG. 15B, respectively. The circle specified by 1402 indicates the location of a defect (e.g., septum of a septal defect) relative to the (transitioned or standard) braided occluder when deployed at a target site. The proximal disc has better coverage of defect 1402 for the transitioned device (right), as shown in FIG. 15C, since an outer edge of the proximal disc on the transitioned device extends farther past defect 1402 than the proximal disc on the standard device. In other words, a distance 1404 from defect 1402 to an outer edge of the proximal disc for transitioned device (right) is larger than a distance 1406 from defect 1402 to an outer edge of the proximal disc for the standard device (left). Better proximal disc coverage is attributable to the waist of transitioned device (right) being better able to fill more of defect 1402 than the standard device (left). Further, more wire in the waist allows for more lengthening and flexibility of the waist section. Benefits of the embodiments described herein include, but are not limited to, increased endothelial growth due to closer contact between the device and atrium(s), increased defect occlusion due to the waist filling out the defect, increased defect occlusion due to decreased bulging which results in increased contact between the disc edges and the atrium(s), increased defect occlusion particularly when the disc patches are sewn directly to the disc edge, and increased embolization resistance.

Conventionally, PPI is relatively high (e.g., 30-40 PPI for 144 wire braid) at the braided diameter (e.g., 40mm), so that the braid does not expand to a diameter much larger than the diameter at which it is braided. As described herein, a much lower PPI (e.g., 20 PPI for 144 wire braid) at a larger diameter (e.g., 50 mm) results in a maximum expanded braid diameter of ∼ 60 mm. The 60 mm maximum expanded diameter braid provides a significantly softer (e.g., radially softer) formed device than the conventional 40 mm braid diameter. Accordingly, for exemplary embodiments of a 26 mm formed device, PPI may range from about 10 PPI to about 55 PPI, and expanded braid diameter may range from about 40 mm to about 60 mm. In some embodiments of the 26 mm formed device, the braided layer has a smaller diameter and/or higher pic rate near the waist and underside (i.e., waist-facing side) of each disc portion, such that the waist and inner disc portions of the device have an expanded diameter between 26 mm and 40 mm, for example.

### b. 144 or 288 Wire Braid

In some embodiments, the medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the at least one braided layer comprises a wire braid design between a 72 wire braid design and a 288 wire braid design. In some embodiments, the medical device compliance on cardiac tissue is increased by lowering a stiffness of the at least one braided layer.

In some embodiments, the use of 144 or 288 wire braid results in the use of lower diameter (softer) wire and more evenly spreads out surface contact forces than, for example, a 72 wire braid by increasing the braid density. 144 or 288 wire braid increases device compliance and reduces friction on the tissue. In particular, increasing the wire count from 72 to 144 (or 288) wire braid necessitates the use of a lower diameter wire in order to keep similar functional properties to other medical devices. By utilizing a 144 or 288 wire braid, the braid opening cell size significantly decreases, allowing for less forceful tissue contact via a greater functional surface area. By allowing lower wire diameters, the compliance of the medical device increases by lowering the wire stiffness.

In some embodiments, the wire braid design comprises from about 12 wire braid to about 288 wire braid and all possible wire braid embodiments in between, including, but not limited to, 12 wire braid, 16 wire braid, 32 wire braid, 36 wire braid, 54 wire braid, 72 wire braid, 96 wire braid, 144 wire braid, or 288 wire braid. In some embodiments, one braided layer (such as an inner layer) comprises a lower wire braid and another braided layer (such as an outer layer) comprises a higher wire braid.

In some embodiments, the at least one braided layer has a wire diameter of from about 0.001 inches to about 0.012 inches.

### c. Multiple Braid Layers with Differing Layer Geometries

In some embodiments, the medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises multiple braided layers, wherein each braided layer comprises a unique layer geometry relative to the other braided layers of the multiple braided layers. In some embodiments, the medical device compliance on cardiac tissue is increased by softening edges of the disc portions and strengthening the waist member.

In some embodiments, adding an additional inner layer of braid with a full-size waist but smaller disc diameters helps strengthen the self-centering mechanism while allowing the discs to remain compliant. In particular, by including multiple layers of braid, with (potentially) different wire diameters, wire counts and separate geometries, they may be used in order to soften the disc edges while strengthening the device waist. With only a single layer of braid at the disc edges, the clamping force exerted near the edge is lower than a device with thinner wires on the outer layer and the disc edge is softer and thus less traumatic to heart tissue.

In some embodiments, a second braided layer provides a second layer to the entire waist member. In other embodiments, a third, fourth, fifth or more braided layers cover the entire waist member, each of differing wire diameters and wire counts. By including in the waist member a second layer the self-centering mechanism may be strengthened without additional stiffening of the discs. With only a single layer of braid at the discs, the clamping force exerted near the disc edge is the same or higher than a device with similar thinner wires but lacking the reinforced waist (due to the stiffer waist pulling the ends toward the center of the device).

In some embodiments, as shown in FIGs. 16A and 16B, the occluder 10 has a configuration that has two layers with different diameter discs, with the inner layer 42 having smaller discs than the outer layer 40 (by necessity). The occluder waist is the same size for both layers. Wire numbers in the braid are varied with additional wires on the outside layer 40 to increase tissue contact surface area and fewer wires on the inside layer 42 to reduce bulk. It is also possible, in some embodiments, to have fewer wires on the outside layer 40 and more wires on the inside layer 42.

In some embodiments, as shown in FIG. 16C, the inner layer 42 braid matches the disc diameters of the outer layer 40, but the waist of the inner layer 42 has a smaller diameter than the outer layer 40. This allows greater independent movement of the discs. Additionally, another embodiment would be for the inner layer to be braided wire and the outer layer to be a braided, knit, woven or non-woven fabric.

In still other embodiments, a combination of the two embodiments shown in FIG. 16D is disclosed. That is, the inner layer 42 has discs and a waist with smaller diameters than the outer layer 40. This provides a softer waist and disc edge than other devices, while providing more clamping force due to the stiff inner discs. Additionally, the soft outer waist moves more easily when external force is exerted on the disc, which allows the entire device to move away from the anatomy that is exerting the external force (such as the aorta), thus reducing the force imparted by the device discs on the anatomy.

FIG. 16E depicts an inner layer 54 surrounding the waist 52 and within the outer layer 50. The inner layer 54 is oval-shaped and the outer layer 50 is configured to be aligned to an aorta/superior area of a septal defect. In some embodiments, the inner layer is oblong-shaped, or any shape similar to that of an oval. In some embodiments, the major diameter of the inner layer extends part or all of the way to the outer braid layer, and the minor diameter of the inner layer extends anywhere from the waist diameter to a location near the outer layer. These embodiments can be orientation-dependent to be aligned to the aorta with the delivery system, or deployed into the left or right atrium for alignment before recapturing the device, aligning it, and redeploying it. In some embodiments, this is achieved using radiopaque markers on portions of the device to indicate the direction of alignment.

In all of the above-mentioned embodiments, the wire diameters also may vary within each layer and between layers, and the wire counts may vary between layers.

Modification of the braid geometry is also achieved in the waist of the device through braiding/forming the braid to increase the radial strength. In some embodiments, radial strength in the waist is increased by setting the braid pic rate (i.e., pics per inch or PPI) to a maximum PPI (relative to an overall desired size for the device) in order to decrease the helix length as much as possible (and therefore increase radial/hoop strength of the waist section). In embodiments when the braid PPI is increased beyond what is considered the maximum PPI for the desired size for the device, the braid at the waist portion bunches up on itself like an accordion, and will thus be configured similarly to that shown in FIGs. 12A-B. Specifically, the pic rate at a center portion 1102 (e.g., a waist portion) of the braid is higher than a pic rate at an outer portion 1104 of the braid pattern located distally and proximally to the center/waist portion 1102 (e.g., distal and proximal discs). Similar to FIG. 12A, the higher pic rate at center portion 1102 causes the braid to bunch up on itself and increase the radial strength at the center/waist portion 1102. For clarity, FIG. 12A shows discernable boundaries between portions of differing pic rates, such as between center portion 1102 and outer portions 1104. In some bunched braid embodiments where pic rates are high and braids are very dense, there are no discernable boundaries between portions having differing pic rates. Moreover, these portions of differing pic rates are enlarged in FIGs. 12A and 12B for clarity. It should be readily understood that in some implementations, the braid pattern may be sufficiently dense such that no braid definition is discernible in one or more of the portions having a higher pic rate.

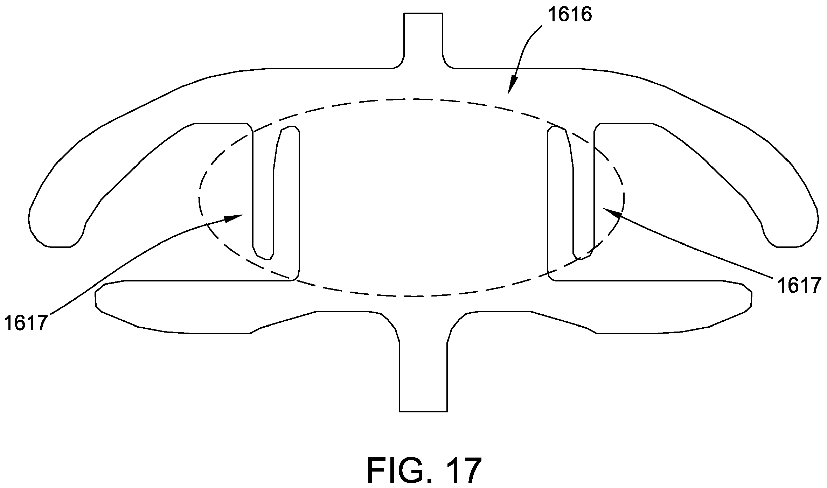

This braiding technique may be limited to certain braid pattern/wire quantity embodiments, as it results in braid that requires more force to elongate, which ultimately increases the radial strength. In other embodiments, modification of the braid geometry is achieved by forcing the braid into a similar configuration through a forming/heat setting process, and/or by braiding onto a mandrel with an uneven surface profile (e.g., a collapsible mandrel that allows removal of the finished braid). In still other embodiments, modification of the braid geometry is achieved by adding a fold 1617 into the braid at the waist section 1616, as shown in FIG. 17, which increases radial force in the waist.

### d. Varying Braid Wire Thickness Through Material Removal

In some embodiments, the medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer with material removed from a portion thereof, wherein the portion of the braided layer with material removed comprises a smaller braid wire diameter at the proximal disc portion and the distal disc portion than at the waist member. In some embodiments, the medical device compliance on cardiac tissue is increased while maintaining a self-centering strength of the medical device.

In some embodiments, electropolishing the distal and proximal discs (without polishing the waist) creates a lower braid wire diameter in a localized region of the disc while maintaining the wire diameter on the waist. This allows increased disc compliance while maintaining the self-centering mechanism's strength. In some embodiments, however, the waist member is not electropolished.

In some embodiments, the braid wire diameter at the proximal disc portion is from about 0.001 inches to about 0.012 inches. In some embodiments, the braid wire diameter at the distal disc portion is from about 0.001 inches to about 0.012 inches. In some embodiments, the braid wire diameter at the waist member is from about 0.001 inches to about 0.012 inches.

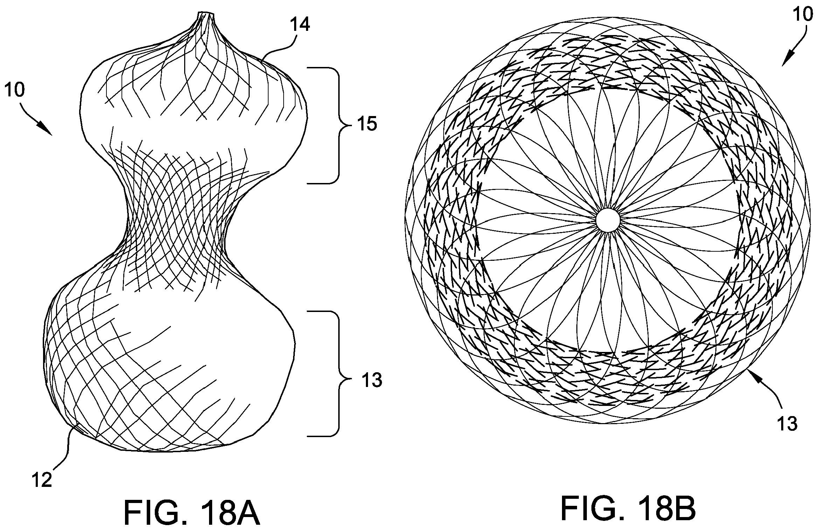

In some embodiments, varying the braid wire thickness through targeted material removal (microblasting, acid, electropolishing, or some combination thereof) reduces the forces exerted by portions of the device (the edge of the left atrial disc 12 and/or the right atrial disc 14) while maintaining strength of other parts of the device (the radial force of the waist member 16 and interior portions of the discs 12, 14 for self-centering, and clamp force/embolization resistance). The amount of material removal depends on the required reduction of force exerted by the edge of the disc 12, 14. For example, if each braid wire starts at .007 inches in diameter, removing material from a portion 13, 15 of the discs 12, 14 until the wire diameter is .002 inches at the edges of the discs significantly reduces the force exerted on the anatomy after implanting the device 10 (see, e.g., FIGs. 18A and 18B). In addition, depending on how the device is masked during manufacturing, the material removal is focused on discrete areas, or, in some embodiments, is a gradual transition.

Material removal can be performed from the ends of the device to an area near the waist, or it can be performed at the edges of the discs only, after the device is formed; this decision will be based on manufacturability and the force requirements of the device.

### e. Independent Waists

In some embodiments, the medical device comprises a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site, wherein the tubular member further comprises a proximal transition segment and a distal transition segment, wherein the proximal transition segment connects the proximal disc portion to the waist member and the distal transition segment connects the distal disc portion to the waist member, and further wherein each of the proximal transition segment and the distal transition segment has a smaller diameter than the waist member. In some embodiments, the medical device compliance on cardiac tissue is increased through greater transitional movement of the disc portions relative to the waist member.

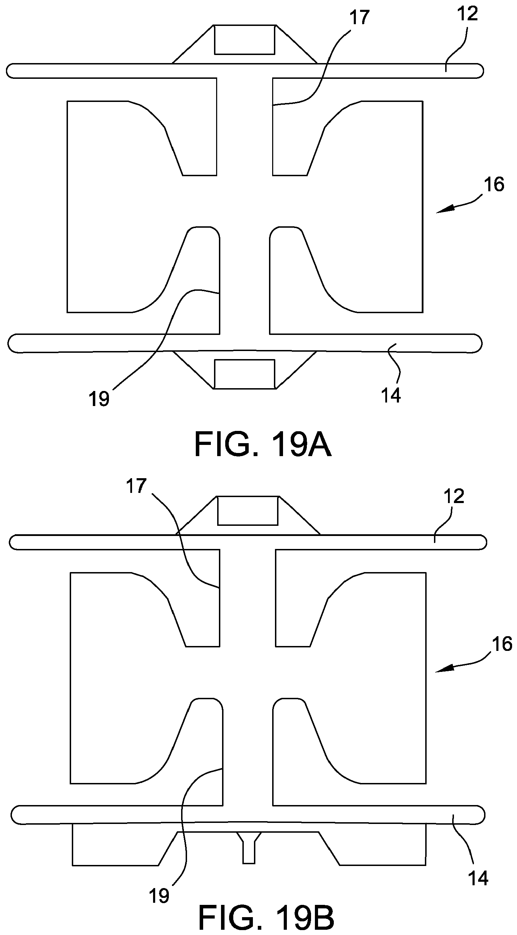

By allowing a thinner connecting section between each disc 12, 14 and the waist member 16, greater disc mobility is achieved by allowing more disc motion relative to the waist member 16 than is allowed under standard medical devices. In particular, shaping the braid to have discs 12, 14 connected to the waist member at transition segments 17, 19 having a much smaller profile, allows significantly greater translational movement of the discs 12, 14 relative to the waist member 16, and allows the discs 12, 14 to shift up against a cardiac structure (e.g., wall, aorta) (see, e.g., FIGs. 19A and 19B).

In some embodiments, the diameter of the proximal transition segment 19 is from about 1 mm to about 5 mm. In some embodiments, the diameter of the distal transition segment 17 is from about 1 mm to about 5 mm. In some embodiments, the waist member 16 has a diameter of from about 2 mm to about 60 mm.

### f. Disc Edge Shape

In some embodiments, the medical device comprises a tubular member comprising a proximal disc portion at a proximal end, a distal disc portion at a distal end, and a waist member extending between the proximal disc portion and the distal disc portion, wherein the proximal disc portion and the distal disc portion comprise an edge geometry selected from the group consisting of a tapered shape, a cup shape, and a round shape, and further wherein the tubular member comprises at least one braided layer and has an expanded configuration when deployed at the target site and a reduced configuration for delivery to the target site. In some embodiments, the medical device compliance on cardiac tissue is increased by deflecting compression forces away from the center of the medical device.

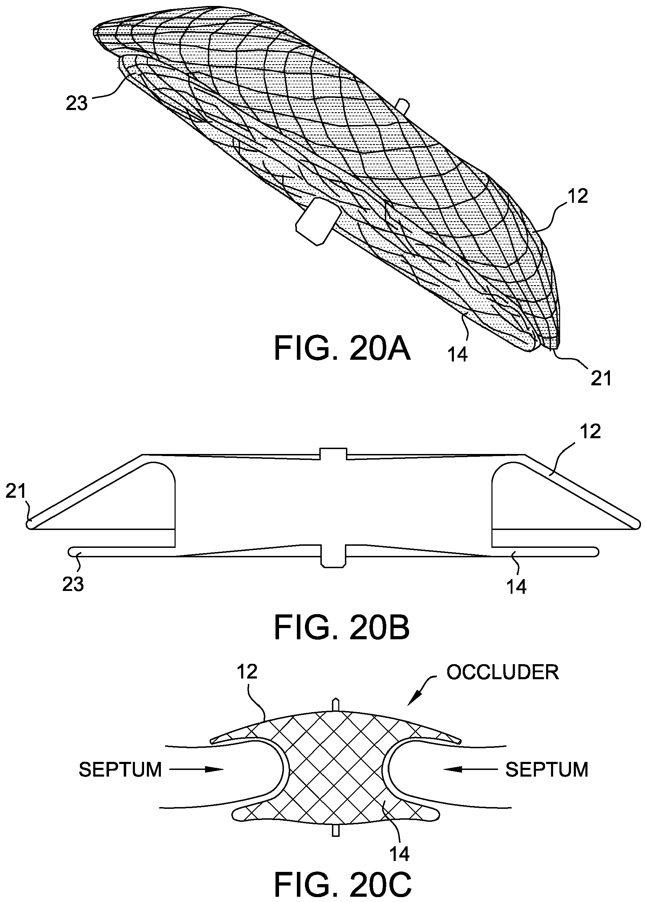

FIGs. 20A, 20B and 20C are exemplary embodiments of an occluder shape configuration in the industry. By rounding an edge 21 and/or 23 of the disc 12 and/or 14, utilizing a modified contact angle, creating a tapered shape or other (e.g., a cup shape), the interaction of the disc(s) 12, 14 with the cardiac tissue significantly improves with more compliant edges 21, 23. In particular, forming different edge geometries on the medical device provides significant benefits in reducing compression forces on the tissue by spreading them over a wider area or allowing the device to accommodate dynamic anatomical movements.

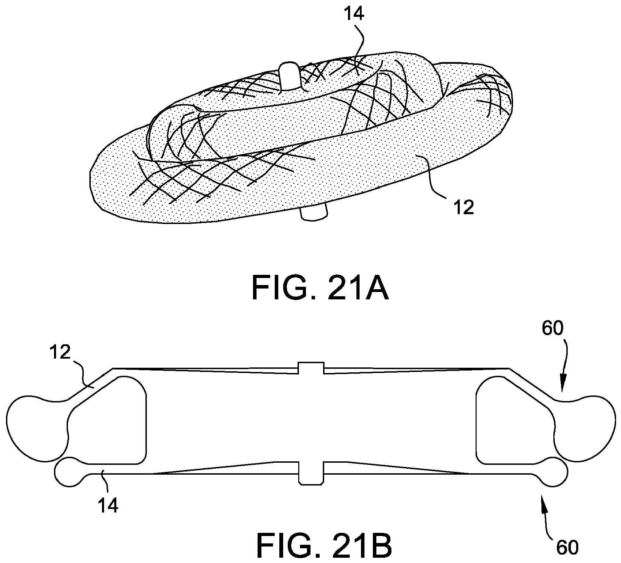

In accordance with the present disclosure, in some embodiments, at least one of the proximal disc portion 14 and the distal disc portion 12 comprise an edge geometry consisting of a round shape 60 (see, e.g., FIGs. 21A and 21B). A rounded edge 60 more evenly spreads the device's wires against an adjacent compressing tissue, and, further, does not create a sharp edge where the outermost part of the disc contacts tissue.

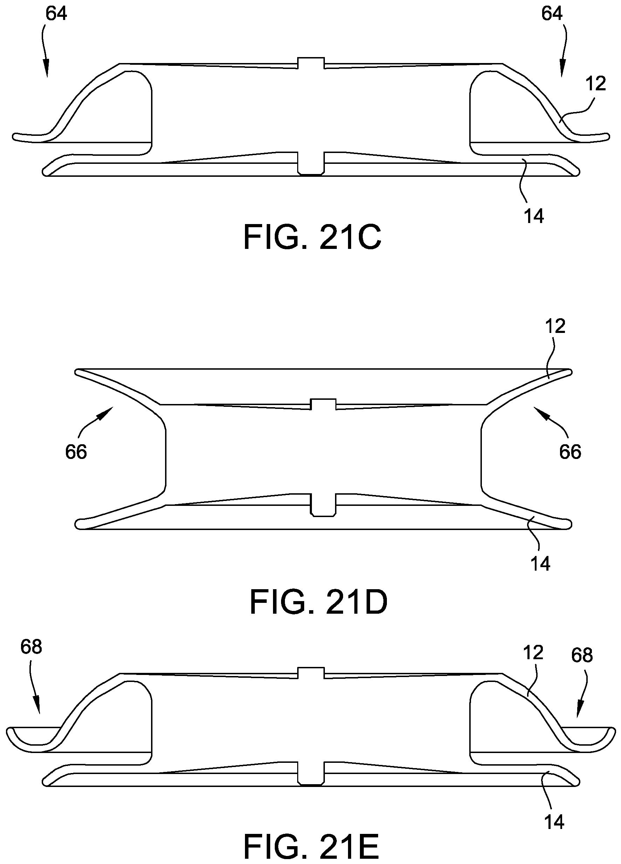

In some embodiments, at least one of the proximal disc portion 14 and the distal disc portion 12 comprise an edge geometry consisting of a tapered shape 64 (see, e.g., FIG. 21C). A tapered shape edge 64 reduces the risk of the device directly protruding into the atrial or aortic structures by deflecting compression forces away from the disc 12, 14 or disc centers.

In some embodiments, at least one of the proximal disc portion 14 and the distal disc portion 12 comprise an edge geometry consisting of an hourglass-shape 66 (see, e.g., FIG. 21D). The hourglass-shape 66 keeps the discs 12, 14 away from the cardiac tissue.

In some embodiments, at least one of the proximal disc portion 14 and the distal disc portion 12 comprise an edge geometry consisting of a cup shape 68 (see, e.g., FIG. 21E). A cup shape edge 68 reduces the risk of the device directly protruding into the atrial or aortic structures by deflecting compression forces away from the disc 12, 14 or disc centers.

### g. Non-Circular Braid Design

In some embodiments, the medical device comprises a tubular member comprising a proximal disc portion at a proximal end, a distal disc portion at a distal end, and a waist member extending between the proximal disc portion and the distal disc portion, wherein the tubular member comprises at least one braided layer comprises a non-circular braid design, and wherein the tubular member has an expanded configuration when deployed at the target site and a reduced configuration for delivery to the target site. In some embodiments, the medical device compliance on cardiac tissue is increased by the medical device avoiding high risk areas of the cardiac anatomy.

By changing the medical device (e.g., occluder) braid design to be other than circular, the high risk areas of the cardiac anatomy (the superior rim and aortic rim of the ASD) are avoided altogether to prevent erosions. In particular, the high risk areas of the superior and aortic rims are avoided while still providing a disc or discs of significant enough strength to prevent embolization.

In some embodiments, as shown in FIG. 22A, the device 10 has a braid design in the shape of an oval. In some embodiments, as shown in FIG. 22B, the braid design is circular with discs 12, 14 offset from the waist member 16. In some embodiments, as shown in FIG. 22C, the device has a braid design in an irregular kidney shape.

In some embodiments, as shown in FIG. 22B, the device 10 has discs 12, 14 that are offset and extend more in one direction.

In some embodiments, as shown in FIG. 22C, the device 10 has a braid design that comes in (e.g., is radially concave towards a waist member, not shown in FIG. 22C) on a side that is towards the aorta so that the device 10 can go around or saddle the aorta.

### h. Standardizing Disc Force

In some embodiments, the medical device comprises a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer, wherein the braided layer comprises multiple wire sizes. In some embodiments, the medical device compliance on cardiac tissue is increased by standardizing the forces of the medical device on the cardiac tissue.

Standard occluders have a range of pull through forces, push through forces and edge compression forces due to only a few different wires used to build all of the occluder sizes. Some of the embodiments of the present disclosure utilize additional wire sizes in conjunction with hybrid braids (multiple wires sizes braided together to attain intermediate characteristics) to attain a single disc pull through force, push through force and/or edge compression force. In particular, forces spike each time an occluder wire size increases .001 inches in thickness, so in order to smooth any occluder force related characteristics, the new braid configurations of the present disclosure increase compliance to an acceptable level with various occluder designs and ease transitions between wire sizes.

Some occluding devices have wire diameter increases from .004 inches to .008 inches across the range of device sizes (4 mm - 40 mm). As the devices get larger, the wire diameter must also get larger to achieve the necessary resistance to embolization. At times, the wire size increases in .001 inch increments, which creates device sizes that are stiffer than others. Utilizing hybrid braids with two different wire sizes, and wire diameters in .0005 inch increments, helps normalize the force across the range of device sizes. Normalizing this force also lowers the stiffness of the braid at the edge of the discs, which reduces the risk of erosion.

In some embodiments, a first wire size is from about 0.001 inches to about 0.012 inches. In some embodiments, a second wire size is from about 0.001 inches to about 0.012 inches.

### i. Disc Profile

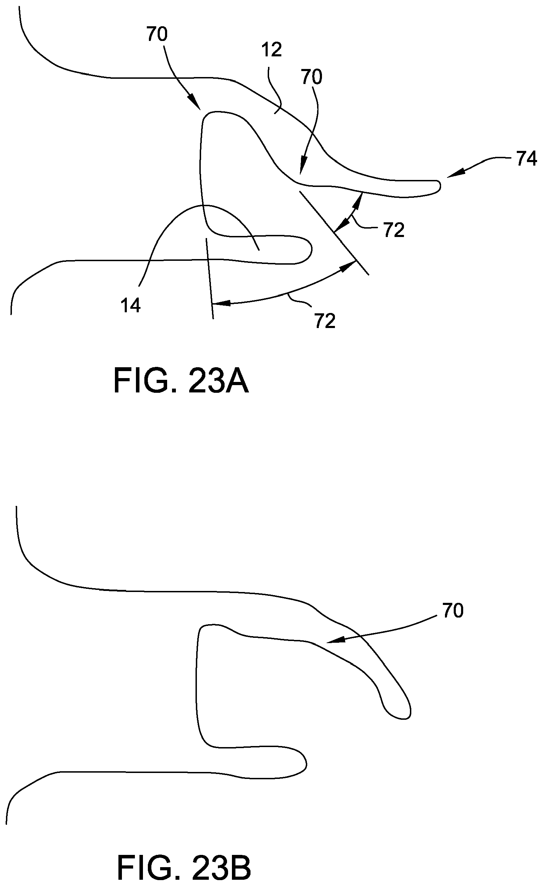

The profile of the disc as it transitions from waist to disc (e.g., including radius and taper angles) affects the clamping forces exerted thereby, and the conformability of the discs. Adjusting this profile provides additional ways of decreasing the braid wire diameters used while maintaining the shape and clamping forces of the device and disc during and after deployment. A few examples, while not all encompassing, are shown in FIGs. 23A and 23B (this configuration applies to both discs 12, 14). FIG. 23A depicts different radii 70 (e.g., measured at different radial locations of disc 12, 14) and different taper angles 72 (e.g., measured relative to a previous angular orientation radially inward therefrom), as well as the disc edge shape 74. Another radius 70 (e.g., measured at a different radial location) is also shown in FIG. 23B. In some embodiments, a profile of the disc (e.g., proximal disc, distal disc, or both) is flat, flat with a tapered edge, flat with a slightly tapered edge, or flat with a cup-shaped edge. In embodiments of devices having more than one disc, the discs may each have the same profile or different profiles.

### j. Termination Profile

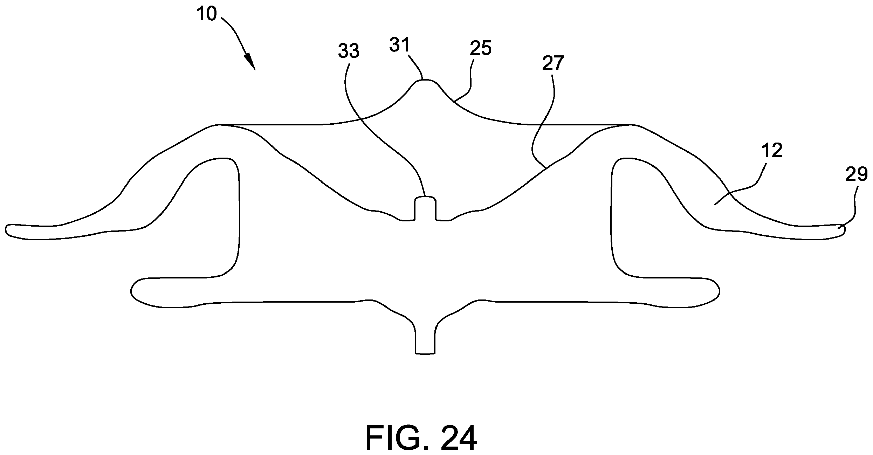

The termination point of the braid and the profile it takes from the discs to each end of the device can be modified to optimize the deployed device profile, and clamping forces of the discs. An example is shown in FIG. 24 (this applies to both discs), depicting two exemplary braid angles or profiles 25, 27 from an edge 29 of the disc 12 to a respective termination point 31, 33 affects disc flexibility, and can be modified to increase/decrease clamping force exerted by the disc 12 and/or device 10 overall.

### Occluding Device Including A Skirt

In some embodiments of the present disclosure, the occluding device includes an external skirt for sealing and cushioning. Structural heart occluders (herein referred to as occluders) are utilized to seal clinically undesirable holes, vascular connections, and appendages within the heart and vasculature, such as an atrial septal defect (ASDs), a patent foramen ovale (PFO), a ventricular septal defect (VSD), a left atrial appendage (LAA), a paravalvular leak channel (PVL), a patent ductus arteriosus (PDA), or an anomalous vascular malformation (AVM). The ASD, PFO, VSD, PVL, PDA, and AVM occluders have a central waist along with two retention discs, while the LAA occluder has a lobe with one disc. To ensure adequate sealing and retention of the occluder, an occluder with a size larger than the structure being occluded is selected for implant. However, usage of a larger size device may sometimes result in complications due to interference with other structures, such as device erosion, heart block, and valvular dysfunction. At times, physicians may elect to implant a smaller device size to avoid these complications and may subsequently have non-optimal sealing. Therefore, there is an unmet need for having an occluder that may provide better sealing without interfering with other structures.

Specific unmet needs for the various occluders include the following:
ASD Occluder - The occluder size selected must adequately seal the ASD; however, if a large device size is implanted; then the retention discs may erode through the atrial free wall into the aorta and cause life-threatening bleeding into the pericardial space requiring emergency surgical intervention.
Post-Infarct VSD Occluder - A VSD formed following a myocardial infarction is not necessarily circular and the tissue along the borders of the VSD are likely to be necrotic. If the implanted device size is not sufficiently large a residual leak will develop, which will prevent the patient from being able to recover. However, if a large device is implanted, then the device will exert pressure on the borders of the VSD and may cause additional tissue necrosis with expansion of the VSD.
Membranous VSD Occluder - A VSD in the membranous septum is challenging to seal because an adequately sized device may exert pressure on the electrical conduction system of the heart and cause heart block with the need to implant a pacemaker.
LAA Occluder - The LAA may not have necessarily a circular cross section such that a larger device size may be needed to adequately seal the LAA. If the device size implanted is large and exerts significant pressure on the LAA walls, the retention wires may cause larger perforations of the LAA with more bleeding into the pericardial space. If the device size implanted is too small, then a residual leak may be present which may also result in formation of a device thrombus and increase the risk for thromboembolic complications.

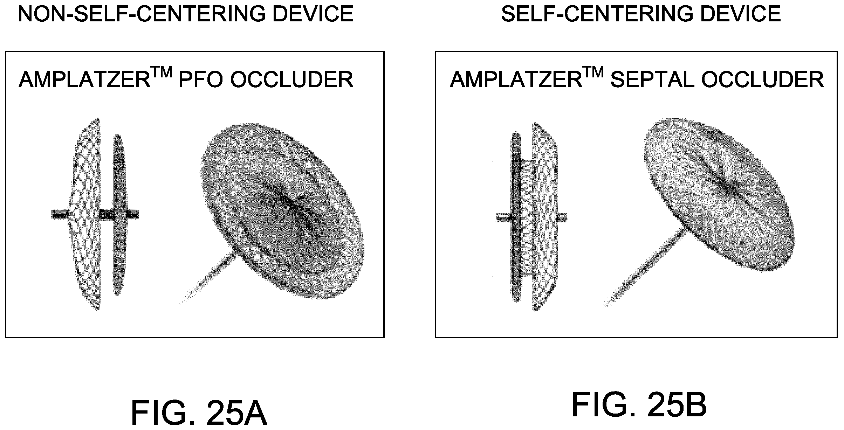

Occluders may be made of a braided nitinol wire mesh that may easily be collapsed and delivered via a catheter. The braid may be made of multiple layers with various calibers of wires to influence occlusion time and device stiffness. The occluder sometimes may also contain an internal fabric material such as polyester to promote occlusion. The central waist of the occluder may be sized to match the size of the defects or may be smaller than the defect size. There are two current designs used for the central waist: (1) A narrow central waist that is not intended to fill the entire defect and which allows the device to freely move within the defect - this design is referred to as non-self-centering (FIG. 25A); and (2) a wider central waist that is sized to completely fill the defect which causes the device to remain in a fixed position centered within the defect- this design is referred to as self-centering (FIG. 25B).

In some embodiments, the central waist of the occluder is designed to be smaller in size (diameter) relative to the size (diameter) of the defect/LAA and an external skirt is added to the central waist which provides improved sealing and cushioning. The skirt may be made from either synthetic material (e.g., polyester fabric) or preserved tissue (e.g., bovine or porcine pericardium), but may also be made of a fine soft nitinol braid. The skirt has the benefit that it may more easily conform into an irregular shaped defect (e.g., non-circular defect) and allows the use of a smaller diameter central waist. The skirt provides improved sealing and serves as a protective cushion from the central waist. With improved sealing the retention disc size may be optimized to minimize interferences with other structures.

The following table (Table 1) provides a list of the device, unmet needs and solutions in accordance with the present disclosure:

**Table 1**

| Device | Unmet Need | Solution |
|---|---|---|
| ASD Occluder | Erosion | Use of a smaller diameter waist which does not fill the defect allows the device to |
| | | become non-self-centering, so the device is pushed away from the aorta and other cardiac structures rather than remain fixed in position. The skirt provides the needed sealing in the presence of a smaller diameter central waist. |
| Post-Infarct VSD Occluder | Residual Leak | Use of a smaller diameter waist reduces the pressure on the necrotic VSD borders and the skirt allows for better sealing in the presence of a non-circular defect. |
| Membranous VSD Occluder | Heart Block | Use of a smaller diameter central waist reduces the pressure on the electrical conduction system of the heart and the skirt allows for adequate sealing when utilizing a smaller central waist. |
| LAA Occluder | Residual Leak; Micro-Perforation | Use of a smaller diameter lobe reduces the pressure on the LAA wall and the skirt allows for better sealing in the presence of a non-circular defect. |

In some embodiments, a skirt made from synthetic material (such as polyester fabric) or pericardial tissue (such as bovine or porcine pericardium) or a fine soft nitinol braid is used and allows the use of a smaller central waist. The skirt provides improved sealing and serves as a protective cushion from the stiffer metallic central waist. With improved sealing the retention disc size may be further optimized (such as smaller diameter or rounded edges) to minimize interferences with other structures.

In some embodiments, the skirt is attached circumferentially to the external surface of the central waist with sutures or other means (such as bonding). The sutures are placed on the proximal portion of the central waist and the skirt is draped over the remainder portion of the central waist. The skirt diameter is chosen to be significantly larger to provide redundancy and adequate filling around the central waist. The thickness of the skirt is optimized to permit an acceptable collapsed device profile within a delivery catheter.

In some embodiments, a single skirt layer is utilized, but in an alternative embodiment more than one skirt may be used. When using multiple skirts, the skirts may be placed one on top of each other, and/or joined to each other using sutures and may contain a softer material in between (such as Gore-Tex™). Also, the skirt could have a pleated design (folds) to allow reduced profile during delivery and allows sealing after fully deployed.

In some embodiments, the skirt extends over the edge of the disc to create a cushion barrier between the stiffer nitinol braid and the heart wall to further protect against erosion of the heart wall due to rubbing of the nitinol wires. Alternatively, the skirt may be thermally bonded to the occluder or it could be sewn. Also, the skirt could be deposited onto the central waist.

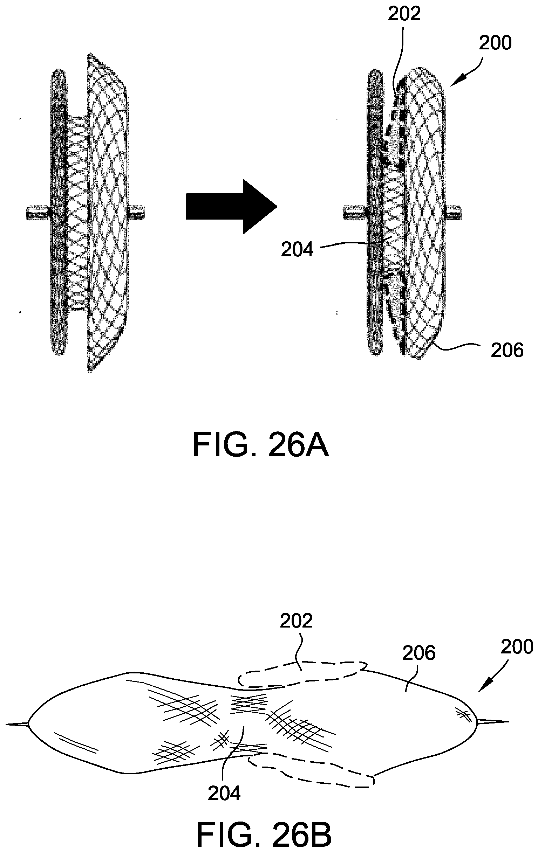

FIG. 26A depicts an occluding device 200 in accordance with the present disclosure, the device 200 including a skirt 202 as described herein. As explained above, the device 200 including the skirt 202 has a relatively smaller central waist 204 (compared to a device without the skirt). FIG. 26B is a profile view of the device 200 with the skirt 202 depicted in stretched form (e.g., for delivery to a target site). In FIGs. 26A and 26B, the skirt 202 is coupled to a distal disc (or lobe) 206 and/or to the central waist 204.

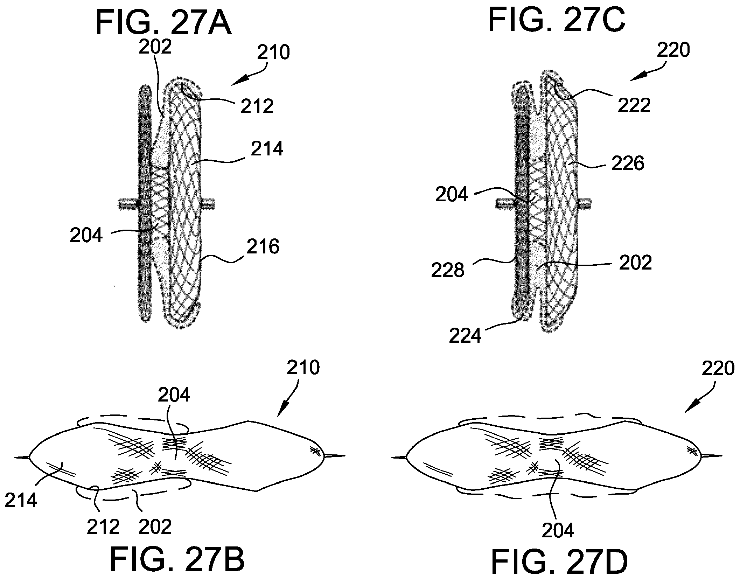

FIG. 27A depicts another embodiment of an occluding device 210 including a skirt 202 covering an edge 212 of a disc 214 (e.g., a left disc). FIG. 27B is a profile view of the device 210 including the skirt 202 in stretched form (e.g., for delivery to a target site). In this embodiment, the skirt 202 is coupled to the disc 214 along the edge 212 of the disc 214 and/or along an end surface 216 of the disc 214. The skirt 202 may also be coupled to the central waist 204.

FIG. 27C depicts another embodiment of an occluding device 220, in which the skirt 202 covers a respective edge 222, 224 of both discs 226, 228. FIG. 27D is a profile view of the device 220 including the skirt 202 in stretched form (e.g., for delivery to a target site). In this embodiment, the skirt 202 is coupled to the disc 226 along the edge 222 thereof and/or is coupled to the disc 228 along the edge 224 thereof.

It is noted that while these embodiments can be applied to various occluder technologies and structures to provide improved sealing and reduced heart block occurrence and are not limited to any one occluder technology.

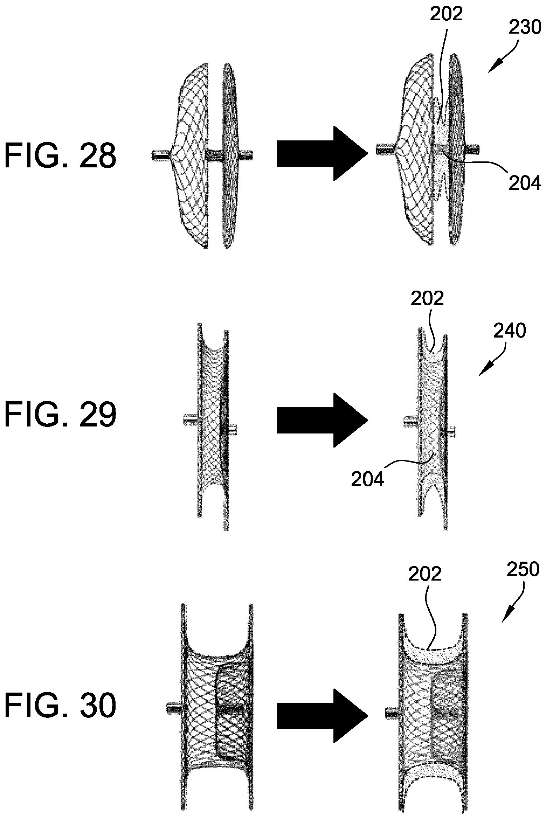

Patent Foramen Ovale (PFO): In some embodiments, as shown in FIG. 28, a sealing skirt 202 is added to a double disc PFO device 230 to prevent a residual leak after implantation. In a certain percentage of PFO cases, there is still a residual leak after implantation due to device placement and/or the anatomical variations of the PFO within the septum. By placing a sealing skirt 202 around the central waist 204 of the PFO device 230, the skirt 202 adapts to the anatomy of the PFO and prevents a residual leak through the PFO tunnel.

Membranous VSD: One of the biggest challenges with a membranous VSD closure is that the outward force from the device on the interventricular septal wall causes electrical disturbances in the heart resulting in heart block. In some embodiments, as shown in FIG. 29, by adding a sealing skirt 202 to the central waist 204 of the membranous VSD device 240, the central waist 204 exhibits less outward force as the sealing skirt 202 provides the necessary sealing between the device 240 and the interventricular septal wall. Often with membranous VSD devices there is difficulty in sealing because the superior rim of the defect is often up against the base of the aortic valve. Besides reducing the occurrence of heart block, this sealing skirt 202 also assists in sealing off superior defects that are challenging to close with occluding devices 240.

Muscular and Post Infarct Muscular VSD: Due to the anatomy of a muscular VSD, especially post-infarct VSDs, there are often challenges with sealing the VSD completely. In some embodiments, as shown in FIG. 30, by applying a sealing skirt 202 to the muscular & post infarct muscular VSD devices 250, it greatly increases the versatility of these devices 250 and reduces residual leaks around the device 250.

### Methods of Using the Device

In accordance with the present disclosure, the medical devices disclosed herein are directed toward methods of eliminating or reducing erosion of cardiac tissue. The methods comprise providing a medical device comprising a tubular member comprising a proximal disc portion at a proximal end and a distal disc portion at a distal end and a waist member extending between the proximal disc portion and the distal disc portion; wherein the tubular member comprises at least one braided layer and has an expanded configuration when deployed at the target site and a reduced configuration for delivery to the target site; constraining the medical device from a preset expanded configuration to a reduced configuration; delivering the medical device; deploying the medical device such that the tubular member returns to the preset expanded configuration; and, eliminating or reducing friction of the medical device on cardiac tissue.

It is understood that each and every embodiment disclosed herein throughout this disclosure is configured to be used according to these methods.

Although certain embodiments of this disclosure have been described above with a certain degree of particularity, those skilled in the art could make numerous alterations to the disclosed embodiments without departing from the spirit or scope of this disclosure. All directional references (e.g., upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of the disclosure. Joinder references (e.g., attached, coupled, connected, and the like) are to be construed broadly and may include intermediate members between a connection of elements and relative movement between elements. As such, joinder references do not necessarily infer that two elements are directly connected and in fixed relation to each other. It is intended that all matter contained in the above description or shown in the accompanying drawings shall be interpreted as illustrative only and not limiting. Changes in detail or structure may be made without departing from the spirit of the disclosure as defined in the appended claims.

When introducing elements of the present disclosure or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions without departing from the scope of the disclosure, it is intended that all matter contained in the above description or shown in the accompanying drawings shall be interpreted as illustrative and not in a limiting sense.

## Claims

1. A medical device for treating a target site, the medical device comprising:
a tubular member comprising a proximal disc portion at a proximal end, a distal disc portion at a distal end, and a waist member extending between the proximal disc portion and the distal disc portion, wherein the tubular member comprises at least one braided layer and has an expanded configuration when deployed at the target site and a reduced configuration for delivery to the target site; and
a coating covering at least a portion of the tubular member.

2. The medical device of claim 1, wherein the coating comprises a fabric coating covering at least one of the proximal disc portion and the distal disc portion.

3. The medical device of claim 1, wherein the coating comprises a polymer coating encapsulating at least a portion of the at least one braided layer.

4. The medical device of claim 1, wherein the coating comprises a parlyene coating covering at least a portion of the at least one braided layer.

5. The medical device of claim 1, wherein the coating comprises a polymeric fabric coating located on an outside surface of the at least one braided layer, wherein the polymeric fabric coating is deposited on the outside surface of the at least one braided layer through an electrospinning process

6. The medical device of claim 1, wherein the coating comprises a ceramic coating on an outside surface of the at least one braided layer.

7. A method of eliminating or reducing erosion of cardiac tissue, the method comprising:
providing a medical device according to claim 1;
constraining the medical device from a preset expanded configuration to a reduced configuration;
delivering the medical device;
deploying the medical device such that the tubular member returns to the preset expanded configuration; and
eliminating or reducing friction of the medical device on cardiac tissue.

8. A medical device for treating a target site, the medical device comprising:
a tubular member comprising a proximal disc portion at a proximal end and a distal disc portion at a distal end and a waist member extending between the proximal disc portion and the distal disc portion, wherein the tubular member has an expanded configuration when deployed at the target site and a reduced configuration for delivery to the target site, and wherein the tubular member comprises multiple braided layers, wherein each braided layer comprises a unique layer geometry relative to the other braided layers of the multiple braided layers.

9. The medical device of claim 8, wherein the multiple braided layers comprise an inner braided layer and an outer braided layer surrounding the inner braided layer, wherein the diameter of the inner braided layer at the proximal disc portion and at the distal disc portion is less than the diameter of the outer braided layer at the proximal disc portion and the distal disc portion, such that an edge of the proximal disc portion and an edge of the distal disc portion of the tubular member only comprise the outer braided layer.

10. The medical device of claim 9, wherein the diameter of the inner braided layer at the waist portion is equal to the diameter of the outer braided layer at the waist portion.

11. The medical device of claim 9, wherein the diameter of the inner braided layer at the waist portion is less than the diameter of the outer braided layer at the waist portion.

12. The medical device of claim 8, wherein the multiple braided layers comprise an inner braided layer and an outer braided layer surrounding the inner braided layer, and wherein a shape of the inner braided layer is different than a shape of the outer braided layer.

13. A medical device for treating a target site, the medical device comprising:
a tubular member comprising a proximal disc portion at a proximal end, a distal disc portion at a distal end, and a waist member extending between the proximal disc portion and the distal disc portion, wherein the tubular member has an expanded configuration when deployed at the target site and a reduced configuration for delivery to the target site, and wherein the tubular member comprises at least one braided layer with material removed from a portion thereof.

14. The medical device of claim 13, wherein the portion of the at least one braided layer with material removed is located at the proximal disc portion and the distal disc portion, such that the at least one braided layer comprises a smaller braid wire diameter at the proximal disc portion and the distal disc portion than at the waist member.

15. The medical device of claim 13, wherein material from the portion of the braided layer is removed using at least one of microblasting, acid, or electropolishing.

16. A medical device for treating a target site, the medical device comprising:
a tubular member comprising a proximal disc portion at a proximal end, a distal disc portion at a distal end, and a waist member extending between the proximal disc portion and the distal disc portion, wherein at least one of the proximal disc portion or the distal disc portion comprise an edge geometry selected from the group consisting of a tapered shape, a cup shape, and a round shape, and wherein the tubular member comprises at least one braided layer and has an expanded configuration when deployed at the target site and a reduced configuration for delivery to the target site.

17. The medical device of claim 16, wherein both of the proximal disc portion and the distal disc portion comprise the edge geometry selected from the group consisting of a tapered shape, a cup shape, and a round shape.

18. The medical device of claim 17, wherein the edge geometry of the proximal disc portion is different from the edge geometry of the distal disc portion.

19. A medical device for treating a target site, the medical device comprising:
a tubular member comprising a proximal disc portion at a proximal end, a distal disc portion at a distal end, a waist member extending between the proximal disc portion and the distal disc portion, wherein the tubular member has an expanded configuration when deployed at the target site and a reduced configuration for delivery to the target site, wherein the tubular member comprises at least one braided layer, wherein a first portion of the at least one braided layer that forms the waist member has a different braid pattern than a second portion of the at least one braided layer forming at least one of the proximal disc portion and the distal disc portion.

20. The medical device of claim 19, wherein the first portion of the at least one braided layer has less pics per inch than the second portion of the at least one braided layer.

21. The medical device of claim 19, wherein the first portion of the at least one braided layer has more pics per inch than the second portion of the at least one braided layer.
